# EUROPÄISCHE PATENTSCHRIFT

(11) **EP 0 862 550 B1**
(45) Veröffentlichungstag und Bekanntmachung des Hinweises auf die Patenterteilung: **08.11.2000**
(21) Anmeldenummer: 96930125.8
(22) Anmeldetag: 29.08.1996
(51) Int. Cl.: C07C 59/64, A61K 31/19, C07C 57/30, C07C 57/38, C07D 257/04, C07C 255/00

(54) **NEUE CARBONSÄUREDERIVATE, IHRE HERSTELLUNG UND VERWENDUNG**
CARBOXYLIC ACID DERIVATIVES, THEIR PREPARATION AND THEIR USE
NOUVEAUX DERIVES D'ACIDE CARBOXYLIQUE, LEUR PREPARATION ET LEUR UTILISATION

(30) Priorität: 07.09.1995 DE 19533025
(43) Veröffentlichungstag der Anmeldung: 09.09.1998
(73) Patentinhaber: BASF AKTIENGESELLSCHAFT, 67056 Ludwigshafen (DE)
(72) Erfinder: AMBERG, Wilhelm, D-61381 Friedrichsdorf (DE); KLING, Andreas, D-68239 Mannheim (DE); KLINGE, Dagmar, D-69120 Heidelberg (DE); RIECHERS, Hartmut, D-67435 Neustadt (DE); UNGER, Liliane, D-67065 Ludwigshafen (DE); RASCHACK, Manfred, D-67256 Weisenheim (DE); HERGENRÖDER, Stefan, D-55128 Mainz (DE); ELGER, Bernd, D-67433 Neustadt (DE); SCHULT, Sabine, D-67346 Speyer (DE)
(86) Internationale Anmeldenummer: EP9603793
(87) Internationale Veröffentlichungsnummer: WO9709294

(56) Entgegenhaltungen:
- WO-A-95/03295

## Beschreibung

Die vorliegende Erfindung betrifft neue Carbonsäuredrivate, deren Herstellung und Verwendung.

Endothelin ist ein aus 21 Aminosäuren aufgebautes Peptid, das von vaskulärem Endothel synthetisiert und freigesetzt wird. Endothelin existiert in drei Isoformen, ET-1, ET-2 und ET-3. Im Folgenden bezeichnet "Endothelin" oder "ET" eine oder alle Isoformen von Endothelin. Endothelin ist ein potenter Vasokonstriktor und hat einen starken Effekt auf den Gefäßtonus. Es ist bekannt, daß diese Vasokonstriktion von der Bindung von Endothelin an seinen Rezeptor verursacht wird (Nature, 332, 411-415, 1988; FEBS Letters, 231, 440-444, 1988 und Biochem. Biophys. Res. Commun., 154, 868-875, 1988).

Erhöhte oder abnormale Freisetzung von Endothelin verursacht eine anhaltende Gefäßkontraktion in peripheren, renalen und zerebralen Blutgefäßen, die zu Krankheiten führen kann. Wie in der Literatur berichtet, wurden erhöhte Plasmaspiegel von Endothelin gefunden bei Patienten mit Hypertonie, akutem Myokardinfarkt, pulmonärer Hypertonie, Raynaud-Syndrom, Atherosklerose und in den Atemwegen von Asthmatikern (Japan J. Hypertension, 12, 79 (1989), J. Vascular Med. Biology 2, 207 (1990), J. Am. Med. Association 264, 2868 (1990)).

Demnach sollten Substanzen, die spezifisch die Bindung von Endothelin an den Rezeptor inhibieren, auch die obengenannten verschiedenen physiologischen Effekte von Endothelin antagonisieren und daher wertvolle Pharmaka darstellen.

Es wurde bereits gefunden, (WO 94/02474), daß bestimmte Carbonsäurederivate mit der allgemeinen Formel Q gute Hemmstoffe für Endothelinrezeptoren sind

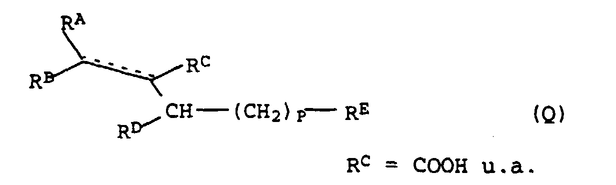

Hierbei werden aber vorwiegend Verbindungen mit einer Doppelbindung im Molekül berücksichtigt. Neben R^{A} und R^{B} wird maximal ein Wasserstoffatom am β-Zentrum zugelassen.

Überraschenderweise wurde nun gefunden, daß dieses Wasserstoffatom durch Alkylreste ersetzt werden kann. Dabei entsteht ein quarternäres β-Zentrum, wodurch gleichzeitig eine große Wirksteigerung gegenüber Endothelinrezeptoren erreicht wird (s. Beispiele).

In WO 95/03295 werden Endothelinantagonisten der allgemeinen Formel (P) beschrieben 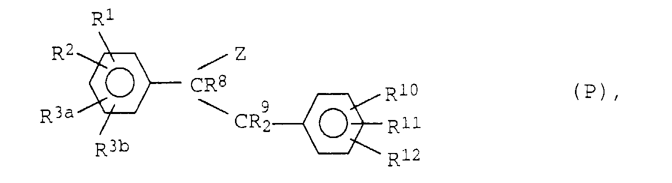 wobei Z = COOH und R⁸ = H sein kann. Die Affinität dieser Substanzen zum Endothelinrezeptor wird nicht angegeben.

Gegenstand der Erfindung sind Carbonsäurederivate der Formel I 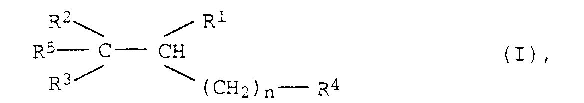 in der R¹ ein Tetrazol, Nitril, eine Gruppe COOH oder einen zu COOH hydrolysierbaren Rest bedeutet und die übrigen Substituenten folgende Bedeutung haben:
- R² und R³: (die gleich oder verschieden sein können):
Phenyl oder Naphthyl, die durch einen oder mehrere der folgenden Reste substituiert sein können: Halogen, Cyano, NO₂, Hydroxy, C₁-C₄-Alkyl, C₁-C₄-Halogenalkyl, C₁-C₄-Alkoxy, C₁-C₄-Halogenalkoxy, Phenoxy, C₁-C₄-Alkylthio, Amino, Benzyloxy, C₁-C₄-Alkylamino oder C₁-C₄-Dialkylamino; oder
Phenyl oder Naphthyl, die orthoständig über eine direkte Bindung, eine Methylen-, Ethylen- oder Ethenylengruppe, ein Sauerstoff- oder Schwefelatom miteinander verbunden sind;
- R⁴: Phenyl oder Naphthyl, Methylendioxyphenyl, Ethylendioxyphenyl, Indanyl, Indolyl, Pyridyl, Benzopyranyl, Furanyl, Benzofuranyl, Isooxazolyl, Isothiazolyl, 1,3,4-Thiadiazolyl, Pyrimidinyl, 2,3-Dihydrobenzofuranyl, Benzothienyl, Chinolinyl, C₃-C₇-Cycloalkyl, Thienyl, Oxazolyl, Thiazolyl, die durch einen oder mehrere der folgenden Reste substituiert sein können: Halogen, Cyano, Hydroxy, NO₂, C₁-C₄-Alkyl, C₁-C₄-Halogenalkyl, C₁-C₄-Alkoxy, C₁-C₄-Halogenalkoxy, Phenoxy, C₁-C₄-Alkylthio, Amino, Benzyloxy, C₁-C₄-Alkylamino oder C₁-C₄-Dialkylamino, wobei die Alkyreste gemeinsam einen Ring bilden können;
- R⁵: C₁-C₈-Alkyl, C₃-C₆-Alkenyl, C₃-C₆-Alkinyl oder C₃-C₈-Cycloalkyl, wobei diese Reste jeweils ein- oder mehrfach substituiert sein können durch: Halogen, C₁-C₄-Alkoxy, C₁-C₄-Alkylthio, C₁-C₄-Alkylamino, Di-C₁-C₄-alkylamino;
Phenyl, Benzyl, 1-Methylnaphthyl, 2-Methylnaphthyl oder Naphthyl, die jeweils durch einen oder mehrere der folgenden Reste substituiert sein können: Halogen, Cyano, Hydroxy,
Amino, C₁-C₄-Alkyl, C₁-C₄-Alkoxy, Phenoxy, C₁-C₄-Alkylthio, Dioxomethylen oder Dioxoethylen;
- n: 1 - 2

Die Verbindungen und auch die Zwischenprodukte zu ihrer Herstellung, wie z.B. Va, können ein oder mehrere asymmetrische substituierte Kohlenstoffatome besitzen. Solche Verbindungen können als reine Enantiomere bzw. reine Diastereomere oder als deren Mischung vorliegen. Bevorzugt ist die Verwendung einer enantiomerenreinen Verbindung als Wirkstoff.

Gegenstand der Erfindung ist weiter die Verwendung der oben genannten Carbonsäurederivate zur Herstellung von Arzneimitteln, insbesondere zur Herstellung von Hemmstoffen für Endothelinrezeptoren.

Verbindungen der Formel I lassen sich herstellen, wenn man zunächst ein Keton vom Typ II mit einem Phosphonoester der Formel III in Gegenwart einer Base zu Verbindungen mit der Formel IV umsetzt.

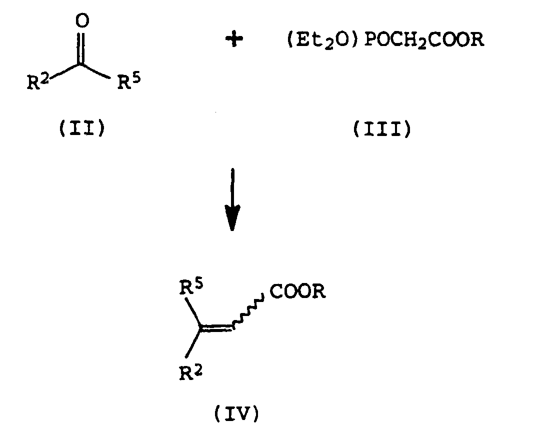

Als Solvens dienen aprotische polare Lösungsmittel wie z.B. DMF oder THF.

Als Base kann ein Alkali- oder Erdalkalimetallhydrid wie Natriumhydrid, Kaliumhydrid oder Calciumhydrid, ein Carbonat wie Alkalimetallcarbonat, z.B. Natrium- oder Kaliumcarbonat, ein Alkali- oder Erdalkalimetallhydroxid wie Natrium- oder Kaliumhydroxid, eine metallorganische Verbindung wie Butyllithium, ein Alkalialkoholat wie Natriumethanolat oder Kaliumtertbutanolat oder ein Alkaliamid wie Lithiumdiisopropylamid dienen.

Die Reaktion wird dabei bevorzugt in einem Temperaturbereich zwischen 0°C und dem Siedepunkt des Lösungsmittels bzw. Lösungsmittelgemisches durchgeführt.

Die Verbindungen vom Typ IV lassen sich dann mit aromatischen Verbindungen in Gegenwart eines Katalysators zu Carbonsäurederivaten mit der allgemeinen Formel Va umsetzen.

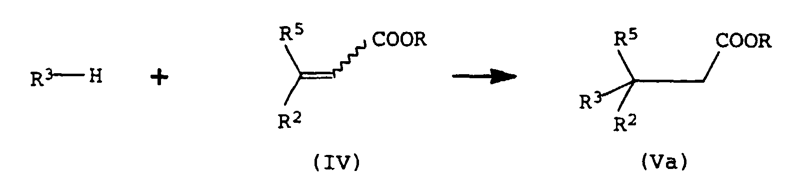

Als Katalysatoren kommen dabei starke anorganische Säuren sowie Lewissäuren in Frage. Beispiele hierfür sind unter anderem Schwefelsäure, Aluminiumtrichlorid, Zinkchlorid oder Eisentrichlorid. Verwendet man Schwefelsäure, so kann die freie Säure direkt erhalten werden.

Alternativ können symmetrische Carbonsäurederivate der Formel Vb aus einer β-Dicarbonylverbindung VI und einer aromatischen Verbindung in Gegenwart eines Katalysators hergestellt werden.

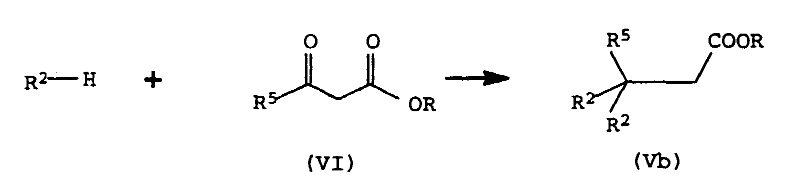

Als Katalysatoren kommen dabei starke anorganische Säuren sowie Lewissäuren in Frage. Beispiele hierfür sind unter anderem Schwefelsäure, Aluminiumtrichlorid, Zinkchlorid oder Eisentrichlorid (siehe auch: Gogte G.R. et al., J. Univ. Bombay, Sect. A, 27, 1958, 41).

Eine weitere Möglichkeit zur Herstellung von Verbindungen des Typs Va kann von einem Keton VII ausgehen, 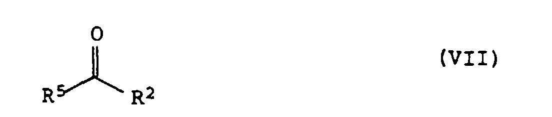 das mit Meldrumsäure in Gegenwart einer Base wie Pyridin oder Natriumhydrid zu Verbindungen vom Typ VIII umgesetzt werden kann

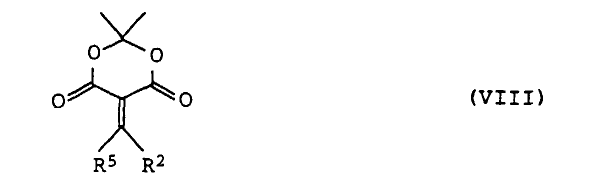

Setzt man Verbindungen vom Typ VIII in Diethylether mit einem Grignard-Reagenz der allgemeinen Formel IX um 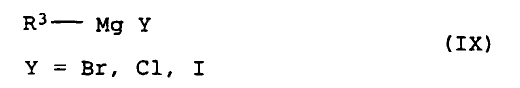 so erhält man Verbindungen vom Typ X, 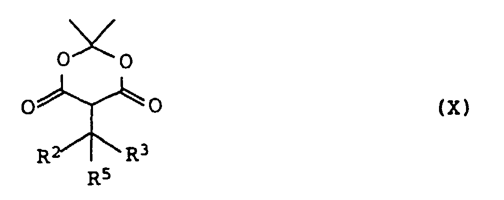 wobei die zusätzliche Verwendung von Kupfersalzen wie Kupferchlorid, Kupferbromid, Kupferiodid oder Kupfercyanid und die Gegenwart einer Lewissäure wie Trimethylsilylchlorid oder Bortrifluoridetherat von Vorteil sein kann.

Die Hydrolyse von Verbindungen der Formel X mit Mineralsäuren wie Salzsäure oder Schwefelsäure kann dann die Verbindungen Va (R = OH) liefern.

Weitere Möglichkeiten zur Herstellung von Verbindungen Va können analog zu Vorschriften von Zimmermann H.E. et al. J. Am. Chem, Soc. 83, 1961, 1196 oder Yu A.J. et al. J. Org. Chem. 23, 1958, 1004 erfolgen.

Verbindungen der Formel Va,b können bei -78°C bis Raumtemperatur mit einer starken Base wie z.B. Butyllithium oder Lithiumdiisopropylamid in einem inerten Lösungsmittel wie z.B. Diethylether oder Tetrahydrofuran und unter Inertgas wie z.B. Stickstoff oder Argon in das Anion (bzw. Dianion für R = H) überführt werden. Dieses Anion reagiert mit Alkylierungsmitteln vom Typ VII bei -78°C bis Raumtemperatur. Nach Quenchen mit konz. NH₄Cl oder verd. Mineralsäure wie HCl erhält man Verbindungen der Formel I

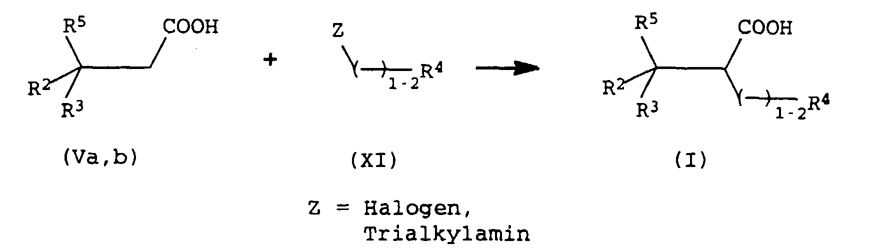

Verbindungen vom Typ I mit R¹ = Tetrazol können ausgehend von den Carbonsäuren I (R¹ = COOH) synthetisiert werden. Dazu wird die Carbonsäure mit Thionylchlorid bei Raumtemperatur zum Säurechlorid und anschließend mit wäßriger Ammoniaklösung zum Säureamid der Formel XII umgesetzt.

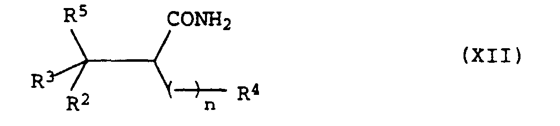

Amide der Formel XII lassen sich mit Oxalylchlorid oder Phosphoroxychlorid oder Trifluoressigsäureanhydrid in DMF oder Pyridin bei 0°C bis Raumtemperatur zu Nitrilen der Formel XIII umsetzen

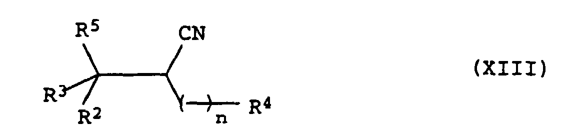

Die Umsetzung von Nitrilen der Formel XIII mit Natriumazid oder Trimethylsilylazid in einem geeigneten Lösungsmittel wie Dimethylformamid, Tetrahydrofuran oder 1-Methyl-2-pyrrolidinon in Gegenwart eines Katalysators wie Ammoniumchlorid (siehe auch: Bernsteim P.R. et al., Synthesis, 1987, 1133) ergibt bei Raumtemperatur oder erhöhter Temperatur die Tetrazole XIV

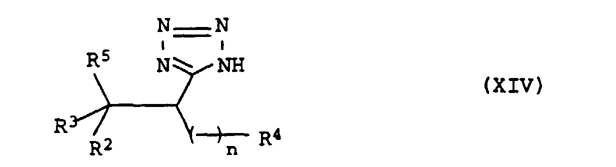

Verbindungen der Formel I können auch dadurch hergestellt werden, daß man von den entsprechenden Carbonsäuren, d. h. Verbindungen der Formel I, in denen R¹ = COOH bedeutet, ausgeht und diese zunächst auf übliche Weise in eine aktivierte Form wie ein Säurehalogenid, ein Anhydrid oder Imidazolid überführt und dieses dann mit einer entsprechenden Hydroxylverbindung HOR umsetzt. Diese Umsetzung läßt sich in den üblichen Lösungsmitteln durchführen und erfordert oft die Zugabe einer Base, wobei die oben genannten in Betracht kommen. Diese beiden Schritte lassen sich beispielsweise auch dadurch vereinfachen, daß man die Carbonsäure in Gegenwart eines wasserabspaltenden Mittels wie eines Carbodiimids auf die Hydroxylverbindung einwirken läßt.

Außerdem können Verbindungen der Formel I auch dadurch hergestellt werden, daß man von den Salzen der entsprechenden Carbonsäuren ausgeht, d. h. von Verbindungen der Formel I, in denen R¹ für eine Gruppe COR und R für OM stehen, wobei M ein Alkalimetallkation oder das Äquivalent eines Erdalkalimetallkations sein kann. Diese Salze lassen sich mit vielen Verbindungen der Formel R-A zur Reaktion bringen, wobei A eine übliche nucleofuge Abgangsgruppe bedeutet, beispielsweise Halogen wie Chlor, Brom, Iod oder gegebenenfalls durch Halogen, Alkyl oder Halogenalkyl substituiertes Aryl- oder Alkylsulfonyl wie z.B. Toluolsulfonyl und Methylsulfonyl oder eine andere äquivalente Abgangsgruppe. Verbindungen der Formel R-A mit einem reaktionsfähigen Substituenten A sind bekannt oder mit dem allgemeinen Fachwissen leicht zu erhalten. Diese Umsetzung läßt sich in den üblichen Lösungsmitteln durchführen und wird vorteilhaft unter Zugabe einer Base, wobei die oben genannten in Betracht kommen, vorgenommen.

Enantiomerenreine Verbindungen der Formel I kann man erhalten, indem man mit racemischen bzw. diastereomeren Verbindungen der Formel VI eine klassische Racematspaltung mit geeigneten enantiomerenreinen Basen wie z.B. Brucin, Strychnin, Chinin, Chinidin, Cinchonidin, Cinchonin, Yohimbin, Morphin, Dehydroabietylamin, Ephedrin (-), (+), Deoxyephedrin (-), (+), threo-2-Amino-1-(p-nitrophenyl)-1,3-propandiol (-), (+), threo-2-(N,N-Dimethylamino)-1-(p-nitrophenyl)-1,3-propandiol (+), (-) threo-2-Amino-1-phenyl-1,3-propandiol (+), (-) α-Methylbenzylamin (+), (-), α-(1-Naphthyl)ethylamin (+), (-), α-(2-Naphthyl)ethylamin (+), (-), Aminomethylpinan, N,N-Dimethyl-1-phenylethylamin, N-Methyl-1-phenylethylamin, 4-Nitrophenylethylamin, Pseudoephedrin, Norephedrin, Norpseudoephedrin, Aminosäurederivate, Peptidderivate durchgeführt.

Der Rest R1 in Formel I ist breit variabel. Beispielsweise steht R¹ für eine Gruppe 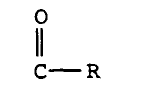 in der R die folgende Bedeutung hat:
a) eine Succinylimidoxygruppe;
b) ein über ein Stickstoffatom verknüpfter 5-gliedriger Heteroaromat wie Pyrrolyl, Pyrazolyl, Imidazolyl und Triazolyl, welcher ein bis zwei Halogenatome, insbesondere Fluor und Chlor und/oder ein bis zwei der folgenden Reste tragen kann:
   C₁-C₄-Alkyl wie Methyl, Ethyl, 1-Propyl, 2-Propyl, 2-Methyl-2-propyl, 2-Methyl-1-propyl, 1-Butyl, 2-Butyl;
   C₁-C₄-Halogenalkyl, insbesondere C₁-C₂-Halogenalkyl wie beispielsweise Fluormethyl, Difluormethyl, Trifluormethyl, Chlordifluormethyl, Dichlorfluormethyl, Trichlormethyl, 1-Fluorethyl, 2-Fluorethyl, 2,2-Difluorethyl, 2,2,2-Trifluorethyl, 2-Chlor-2,2-difluorethyl, 2,2-Dichlor-2-fluorethyl, 2,2,2-Trichlorethyl und Pentafluorethyl;
   C₁-C₄-Halogenalkoxy, insbesondere C₁-C₂-Halogenalkoxy wie Difluormethoxy, Trifluormethoxy, Chlordifluormethoxy, 1-Fluorethoxy, 2-Fluorethoxy, 2,2-Difluorethoxy, 1,1,2,2-Tetrafluorethoxy, 2,2,2-Trifluorethoxy, 2-Chlor-1,1,2-trifluorethoxy und Pentafluorethoxy, insbesondere Trifluormethoxy;
   C₁-C₄-Alkoxy wie Methoxy, Ethoxy, Propoxy, 1-Methylethoxy, Butoxy, 1-Methylpropoxy, 2-Methylpropoxy, 1,1-Dimethylethoxy, insbesondere Methoxy, Ethoxy, 1-Methylethoxy;
   C₁-C₄-Alkylthio wie Methylthio, Ethylthio, Propylthio, 1-Methylethylthio, Butylthio, 1-Methylpropylthio, 2-Methylpropylthio, 1,1-Dimethylethylthio, insbesondere Methylthio und Ethylthio;
c) R ferner ein Rest 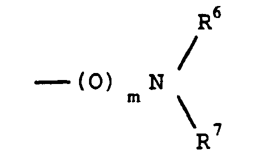 in dem m für 0 oder 1 steht und R⁶ und R⁷, die gleich oder unterschiedlich sein können, die folgende Bedeutung haben:
   Wasserstoff
   C₁-C₈-Alkyl, insbesondere C₁-C₄-Alkyl wie oben genannt; C₃-C₆-Alkenyl wie 2-Propenyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 1,3-Dimethyl-3-butenyl, 2,2-Dimethyl-3-butenyl, 2,3-Dimethyl-2-butenyl, 2,3-Dimethyl-3-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-1-methyl-2-propenyl und 1-Ethyl-2-methyl-2-propenyl, insbesondere 2-Propenyl, 2-Butenyl, 3-Methyl-2-butenyl und 3-Methyl-2-pentenyl;
   C₃-C₆-Alkinyl wie 2-Propinyl, 2-Butinyl, 3-Butinyl, 1-Methyl-2-propinyl, 2-Pentinyl, 3-Pentinyl, 4-Pentinyl, 1-Methyl-3-butinyl, 2-Methyl-3-butinyl, 1-Methyl-2-butinyl, 1,1-Dimethyl-2-propinyl, 1-Ethyl-2-propinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-4-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 1-Ethyl-2-butinyl, 1-Ethyl-3-butinyl, 2-Ethyl-3-butinyl und 1-Ethyl-1-methyl-2-propinyl, vorzugsweise 2-Propinyl, 2-Butinyl, 1-Methyl-2-propinyl und 1-Methyl-2-butinyl, insbesondere 2-Propinyl
   C₃-C₈-Cycloalkyl, wie Cyclopropyl, Cyclobutyl, Cyclopentyl, Cyclohexyl und Cycloheptyl, Cyclooctyl, wobei diese Alkyl-, Cycloalkyl-, Alkenyl- und Alkinylgruppen jeweils ein bis fünf Halogenatome, insbesondere Fluor oder Chlor und/oder ein bis zwei der folgenden Gruppen tragen können:
   C₁-C₄-Alkyl, C₁-C₄-Alkoxy, C₁-C₄-Alkylthio, C₁-C₄-Halogenalkoxy wie vorstehend genannt, C₃-C₆-Alkenyloxy, C₃-C₆-Alkenylthio, C₃-C₆-Alkinyloxy, C₃-C₆-Alkinylthio, wobei die in diesen Resten vorliegenden Alkenyl- und Alkinylbestandteile vorzugsweise den oben genannten Bedeutungen entsprechen;
   C₁-C₄-Alkylcarbonyl wie insbesondere Methylcarbonyl, Ethylcarbonyl, Propylcarbonyl, 1-Methylethylcarbonyl, Butylcarbonyl, 1-Methylpropylcarbonyl, 2-Methylpropylcarbonyl, 1,1-Dimethylethylcarbonyl;
   C₁-C₄-Alkoxycarbonyl wie Methoxycarbonyl, Ethoxycarbonyl, Propyloxycarbonyl, 1-Methylethoxycarbonyl, Butyloxycarbonyl, 1-Methylpropyloxycarbonyl, 2-Methylpropyloxycarbonyl, 1,1-Dimethylethoxycarbonyl;
   C₃-C₆-Alkenylcarbonyl, C₃-C₆-Alkinylcarbonyl, C₃-C₆-Alkenyloxycarbonyl und C₃-C₆-Alkinyloxycarbonyl, wobei die Alkenyl- bzw. Alkinylreste vorzugsweise, wie voranstehend im einzelnen aufgeführt, definiert sind;
   Phenyl, gegebenenfalls ein- oder mehrfach, z.B. ein- bis dreifach substituiert durch Halogen, Nitro, Cyano, C₁-C₄-Alkyl, C₁-C₄-Halogenalkyl, C₁-C₄-Alkoxy, C₁-C₄-Halogenalkoxy oder C₁-C₄-Alkylthio wie beispielsweise 2-Fluorphenyl, 3-Chlorphenyl, 4-Bromphenyl, 2-Methylphenyl, 3-Nitrophenyl, 4-Cyanophenyl, 2-Trifluormethylphenyl, 3-Methoxyphenyl, 4-Trifluorethoxyphenyl, 2-Methylthiophenyl, 2,4-Dichlorphenyl, 2-Methoxy-3-methylphenyl, 2,4-Dimethoxyphenyl, 2-Nitro-5-cyanophenyl, 2,6-Difluorphenyl;
   Di-C₁-C₄-Alkylamino wie insbesondere Dimethylamino, Dipropylamino, N-Propyl-N-methylamino, N-Propyl-N-ethylamino, Diisopropylamino, N-Isopropyl-N-methylamino, N-Isopropyl-N-ethylamino, N-Isopropyl-N-propylamino;
   R₆ und R₇ ferner Phenyl, das durch einen oder mehrere, z.B. ein bis drei der folgenden Reste substituiert sein kann: Halogen, Nitro, Cyano, C₁-C₄-Alkyl, C₁-C₄-Halogenalkyl, C₁-C₄-Alkoxy, C₁-C₄-Haloqenalkoxy oder C₁-C₄-Alkylthio, wie insbesondere oben genannt;
   oder R⁶ und R⁷ bilden gemeinsam eine zu einem Ring geschlossene, optionell substituierte, z.B. durch C₁-C₄-Alkyl substituierte C₄-C₇-Alkylenkette, die ein Heteroatom, ausgewählt aus der Gruppe Sauerstoff, Schwefel oder Stickstoff, enthalten kann wie-(CH₂)₄-,-(CH₂)₅-,-(CH₂)₆-, -(CH₂)₇-, -(CH₂)₂-O-(CH₂)₂-, -CH₂-S-(CH₂)₃-, -(CH₂)₂-O-(CH₂)₃-, -NH-(CH₂)₃-, -CH₂-NH-(CH₂)₂-, -CH₂-CH=CH-CH₂-, -CH=CH-(CH₂)₃-;
d) R ferner eine Gruppe 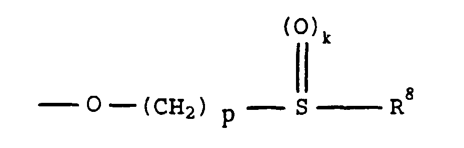 in der k die Werte 0, 1 und 2, p die Werte 1, 2, 3 und 4 annehmen und R⁸ für
   C₁-C₄-Alkyl, C₁-C₄-Halogenalkyl, C₃-C₆-Alkenyl, C₃-C₆-Alkinyl oder gegebenenfalls substituiertes Phenyl steht, wie insbesondere oben genannt.
e) R ferner ein Rest OR⁹, worin R⁹ bedeutet:
   Wasserstoff, das Kation eines Alkalimetalls wie Lithium, Natrium, Kalium oder das Kation eines Erdalkalimetalls wie Calcium, Magnesium und Barium oder ein umweltverträgliches organisches Ammoniumion wie tertiäres C₁-C₄-Alkylammonium oder das Ammoniumion;
   C₃-C₈-Cycloalkyl wie vorstehend genannt, welches ein bis drei C₁-C₄-Alkylgruppen tragen kann;
   C₁-C₈-Alkyl wie insbesonder Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 1,2-Dimethylpropyl, 1,1-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl-2-methylpropyl, welches ein bis fünf Halogenatome, insbesondere Fluor und Chlor und/oder einen der folgenden Reste tragen kann:
   C₁-C₄-Alkoxy, C₁-C₄-Alkylthio, Cyano, C₁-C₄-Alkylcarbonyl, C₃-C₈-Cycloakyl, C₁-C₄-Alkoxycarbonyl, Phenyl, Phenoxy oder Phenylcarbonyl, wobei die aromatischen Reste ihrerseits jeweils ein bis fünf Halogenatome und/oder ein bis drei der folgenden Reste tragen können: Nitro, Cyano, C₁-C₄-Alkyl, C₁-C₄-Halogenalkyl, C₁-C₄-Alkoxy, C₁-C₄-Halogenalkoxy und/oder C₁-C₄-Alkylthio, wie insbesondere oben genannt;
   eine C₁-C₈-Alkylgruppe wie vorstehend genannt, welch ein bis fünf Halogenatome, insbesonder Fluor und/oder Chlor tragen kann und einen der folgenden Reste trägt: ein 5-gliedriger Heteroaromat, enthaltend ein bis drei Stickstoffatome, oder ein 5-gliedriger Heteroaromat enthaltend ein Stickstoffatom und ein Sauerstoff- oder Schwefelatom, welcher ein bis vier Halogenatome und/oder ein bis zwei der folgenden Reste tragen kann:
   Nitro, Cyano, C₁-C₄-Alkyl, C₁-C₄-Halogenalkyl, C₁-C₄-Alkoxy, Phenyl, C₁-C₄-Halogenalkoxy und/oder C₁-C₄-Alkylthio. Insbesondere seien genannt: 1-Pyrazolyl, 3-Methyl-l-pyrazolyl, 4-Methyl-1-pyrazolyl, 3,5-Dimethyl-1-pyrazolyl, 3-Phenyl-1-pyrazolyl, 4-Phenyl-1-pyrazolyl, 4-Chlor-1-pyrazolyl, 4-Brom-1-pyrazolyl, 1-Imidazolyl, 1-Benzimidazolyl, 1,2,4-Triazol-1-yl, 3-Methyl-l,2,4-triazol-l-yl, 5-Methyl-l,2,4-triazol-1-yl, 1-Benztriazolyl, 3-Isopropylisoxazol-5-yl, 3-Methylisoxazol-5-yl, Oxazol-2-yl, Thiazol-2-yl, Imidazol-2-yl, 3-Ethylisoxazol-5-yl, 3-Phenylisoxazol-5-yl, 3-tert.-Butylisoxazol-5-yl;
   eine C₂-C₆-Alkylgrupe, welche in der 2-Position einen der folgenden Reste trägt: C₁-C₄-Alkoxyimino, C₃-C₆-Alkinyloxyimino, C₃-C₆-Halogenalkenyloxyimino oder Benzyloxyimino;
   eine C₃-C₆-Alkenyl- oder eine C₃-C₆-Alkinylgruppe, wobei diese Gruppen ihrerseits ein bis fünf Halogenatome tragen können;
   R⁹ ferner ein Phenylrest, welcher ein bis fünf Halogenatome und/oder ein bis drei der folgenden Reste tragen kann: Nitro, Cyano, C₁-C₄-Alkyl, C₁-C₄-Halogenalkyl, C₁-C₄-Alkoxy, C₁-C₄-Halogenalkoxy und/oder C₁-C₄-Alkylthio, wie insbesondere oben genannt;
   ein über ein Stickstoffatom verknüpfter 5-gliedriger Heteroaromat, enthaltend ein bis drei Stickstoffatome, welcher ein bis zwei Halogenatome und/oder ein bis zwei der folgenden Reste tragen kann: C₁-C₄-Alkyl, C₁-C₄-Halogenalkyl, C₁-C₄-Alkoxy, Phenyl, C₁-C₄-Halogenalkoxy und/oder C₁-C₄-Alkylthio. Insbesondere seien genannt: 1-Pyrazolyl, 3-Methyl-l-pyrazolyl, 4-Methyl-1-pyrazolyl, 3,5-Dimethyl-1-pyrazolyl, 3-Phenyl-l-pyrazolyl, 4-Phenyl-1-pyrazolyl, 4-Chlor-1-pyrazolyl, 4-Brom-1-pyrazolyl, 1-Imidazolyl, 1-Benzimidazolyl, 1,2,4-Triazol-1-yl, 3-Methyl-1,2,4-triazol-1-yl, 5-Methyl-1,2,4-triazol-1-yl, 1-Benztriazolyl, 3,4-Dichlorimidazol-1-yl;
   R⁹ ferner ein Gruppe 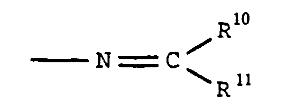 worin R¹⁰ und R¹¹, die gleich oder verschieden sein können, bedeuten:
   C₁-C₈-Alkyl, C₃-C₆-Alkenyl, C₃-C₆-Alkinyl, C₃-C₈-Cycloalkyl, wobei diese Reste einen C₁-C₄-Alkoxy, C₁-C₄-Alkylthio und/oder einen gegebenenfalls substituierten Phenylrest, wie insbesondere vorstehend genannt, tragen können;
   Phenyl, das durch einen oder mehrere, z.B. einen bis drei der folgenden Reste substituiert sein kann: Halogen, Nitro, Cyano, C₁-C₄-Alkyl, C₁-C₄-Halogenalkyl, C₁-C₄-Alkoxy, C₁-C₄-Halogenalkoxy oder C₁-C₄-Alkylthio, wobei diese Reste insbesondere den oben genannten entsprechen;
   oder R¹⁰ und R¹¹ bilden gemeinsam eine C₃-C₁₂-Alkylenkette, welche ein bis drei C₁-C₄-Alkylgruppen tragen und ein Heteroatom aus der Gruppe Sauerstoff, Schwefel und Stickstoff enthalten kann, wie insbesondere bei R⁶ und R⁷ genannt.
f) R ferner ein Rest 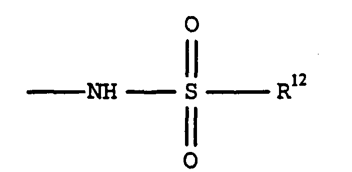 worin R¹² bedeutet:
   C₁-C₄-Alkyl, C₃-C₆-Alkenyl, C₃-C₆-Alkinyl, C₃-C₈-Cycloalkyl wie insbesondere vorstehend genannt, wobei diese Reste einen C₁-C₄-Alkoxy-, C₁-C₄-Alkylthio- und/oder einen Phenylrest wie oben genannt tragen können;
   Phenyl, gegebenenfalls substituiert, insbesondere wie vorstehend genannt.
g) R ein Rest 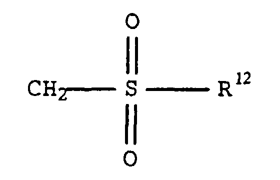 worin R¹² die oben genannte Bedeutung hat.
   - R¹: kann weiterhin sein:
   Tetrazol oder Nitril.
   Im Hinblick auf die biologische Wirkung sind Carbonsäurederivate der allgemeinen Formel I - sowohl als reine Enantiomere bzw. reine Diastereomere als auch als deren Mischung - bevorzugt, in denen die Substituenten folgende Bedeutung haben:
   - R¹: Tetrazol, COOH oder ein zu COOH hydrolysierbarer Rest;
   - R² und R³: (die gleich oder verschieden sein können);
   Phenyl oder Naphthyl, die durch einen oder mehrere der folgenden Reste substituiert sein können: F, Cl, Br, I, Cyano, NO₂, Hydroxy, Methyl, Ethyl, Propyl, Isopropyl, Trifluormethyl, 2,2,2-Trifluorethyl, Methoxy, Ethoxy, Propoxy, Isopropoxy, Trifluormethyloxy, Phenoxy, Methylthio, Ethylthio, Benzyloxy, Amino, Methylamino, Dimethylamino;
   - R⁴: Phenyl, Methylendioxyphenyl, Ethylendioxyphenyl, Indanyl, Pyridyl, 2,3-Dihydrobenzofuranyl, Benzofuranyl, Benzothienyl, 2-Pyrimidinyl, 4-Pyrimidinyl, 2,3-Dihydrobenzothienyl, die durch einen oder mehrere der folgenden Reste substituiert sein können: F, Cl, Br, I, Cyano, NO₂, Methyl, EThyl, Propyl, Isopropyl, Trifluormethyl, Methoxy, Ethoxy, Propoxy, Isopropoxy, Butyloxy, tert.-Butyloxy, Trifluormethyloxy, Phenoxy, Methylthio, Ethylthio, Propylthio, Benzyloxy, Amino, Methylamino, Dimethylamino;
   - R⁵: Methyl, Ethyl, Propyl, Isopropyl, Butyl, 2-Methylpropyl, tert.-Butyl, Pentyl, 3-Methylbutyl, Hexyl, Pent-3-yl, 4-Methylpentyl, 2-Ethylbutyl, die jeweils ein oder mehrfach substituiert sein können durch: Cyano, Methoxy, Ethoxy, Propoxy, Isopropoxy, Butoxy, Methylthio, Ethylthio, Propylthio, Isopropylthio, Amino, Methylamino, Dimethylamino; Allyl, Vinyl, Trifluormethyl, 2,2,2-Trifluorethyl;
   Phenyl, Benzyl, die jeweils durch einen oder mehrere der folgenden Reste substituiert sein können: F, Cl, Br, I, Hydroxy, Methyl, Ethyl, Propyl, Isopropyl, Methoxy, Ethoxy, Propoxy, Isopropoxy, Methylthio, Ethylthio, Dioxomethylen, Dioxoethylen;
   - n: 1 - 2

Beispiele für bevorzugte Verbindungen sind in der folgenden Tabelle ausgeführt:

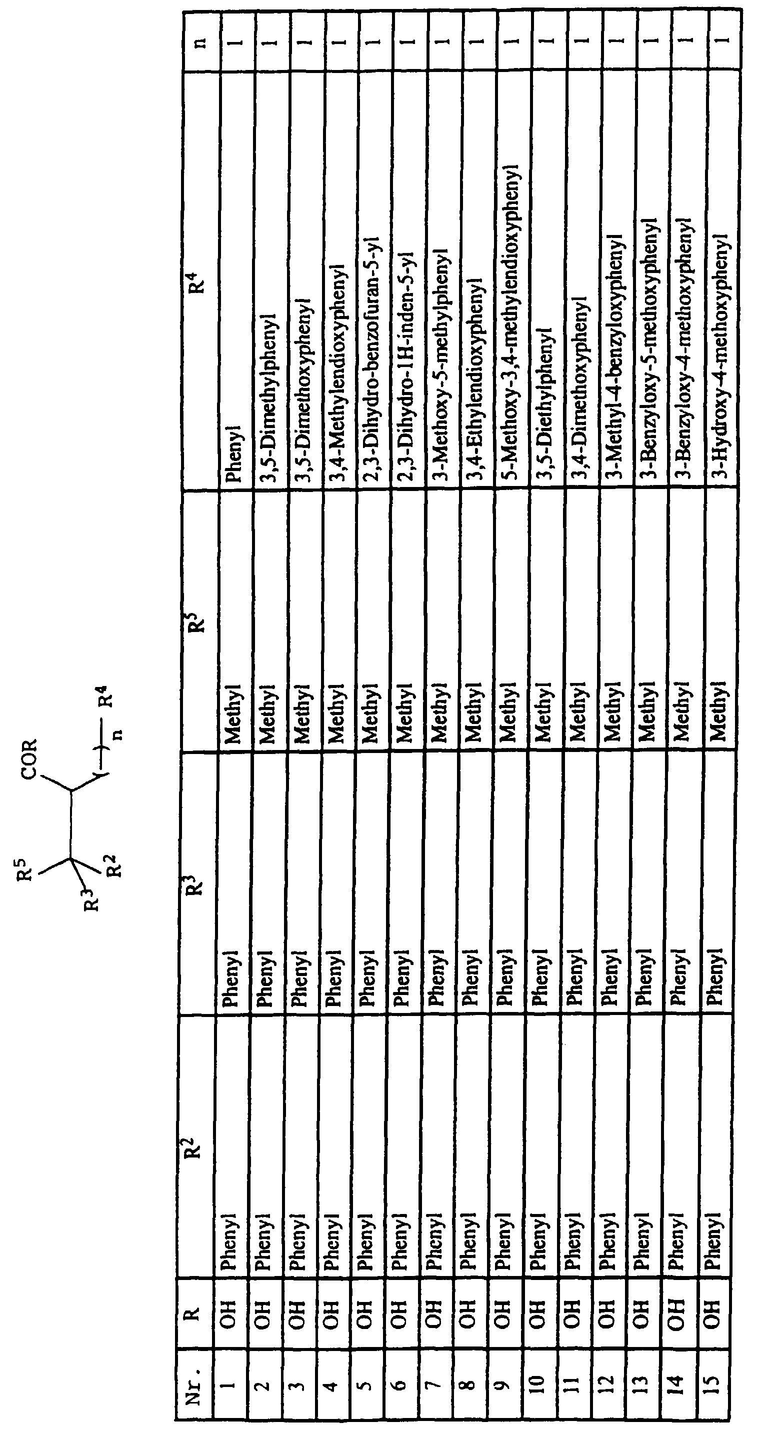

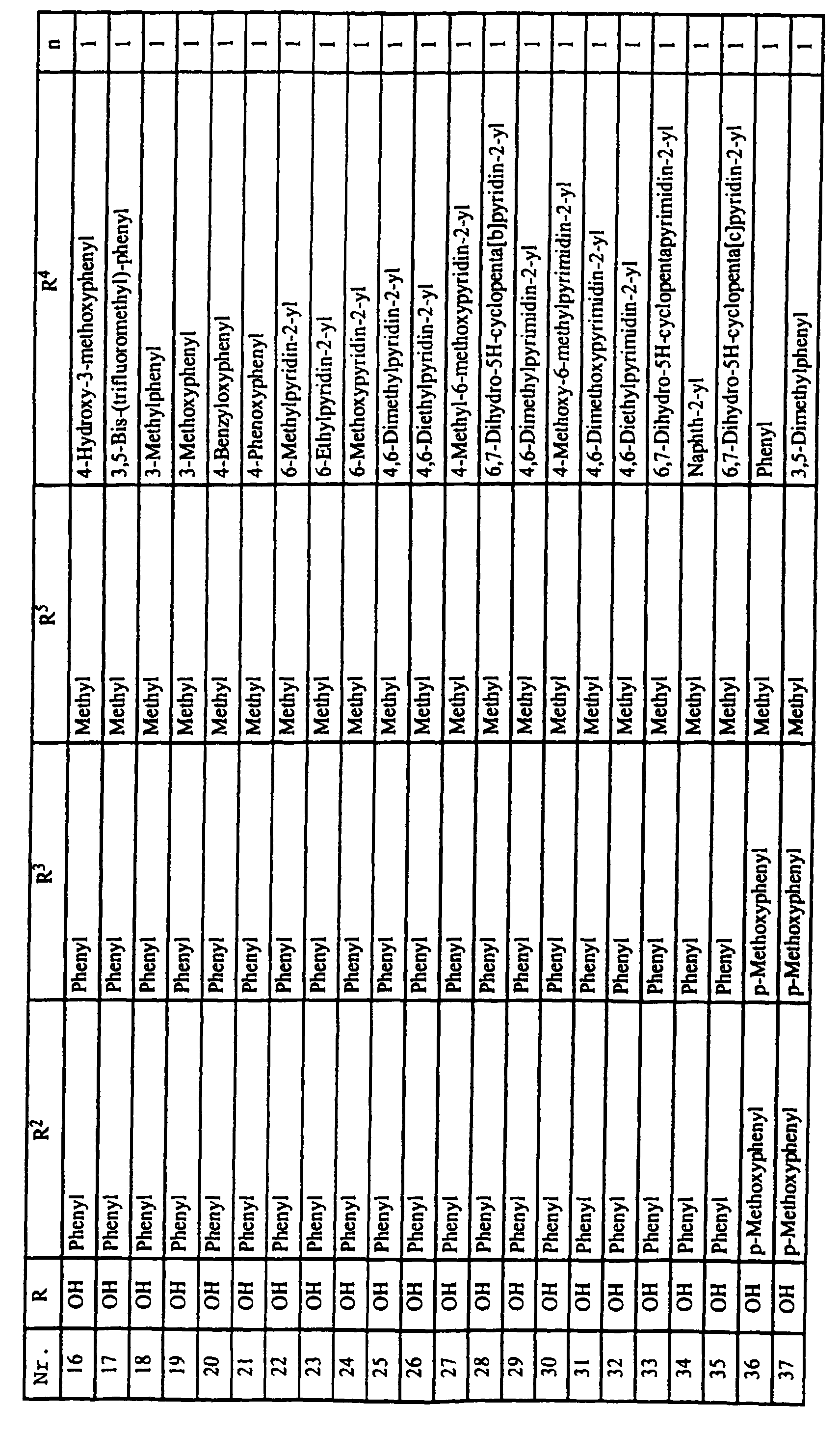

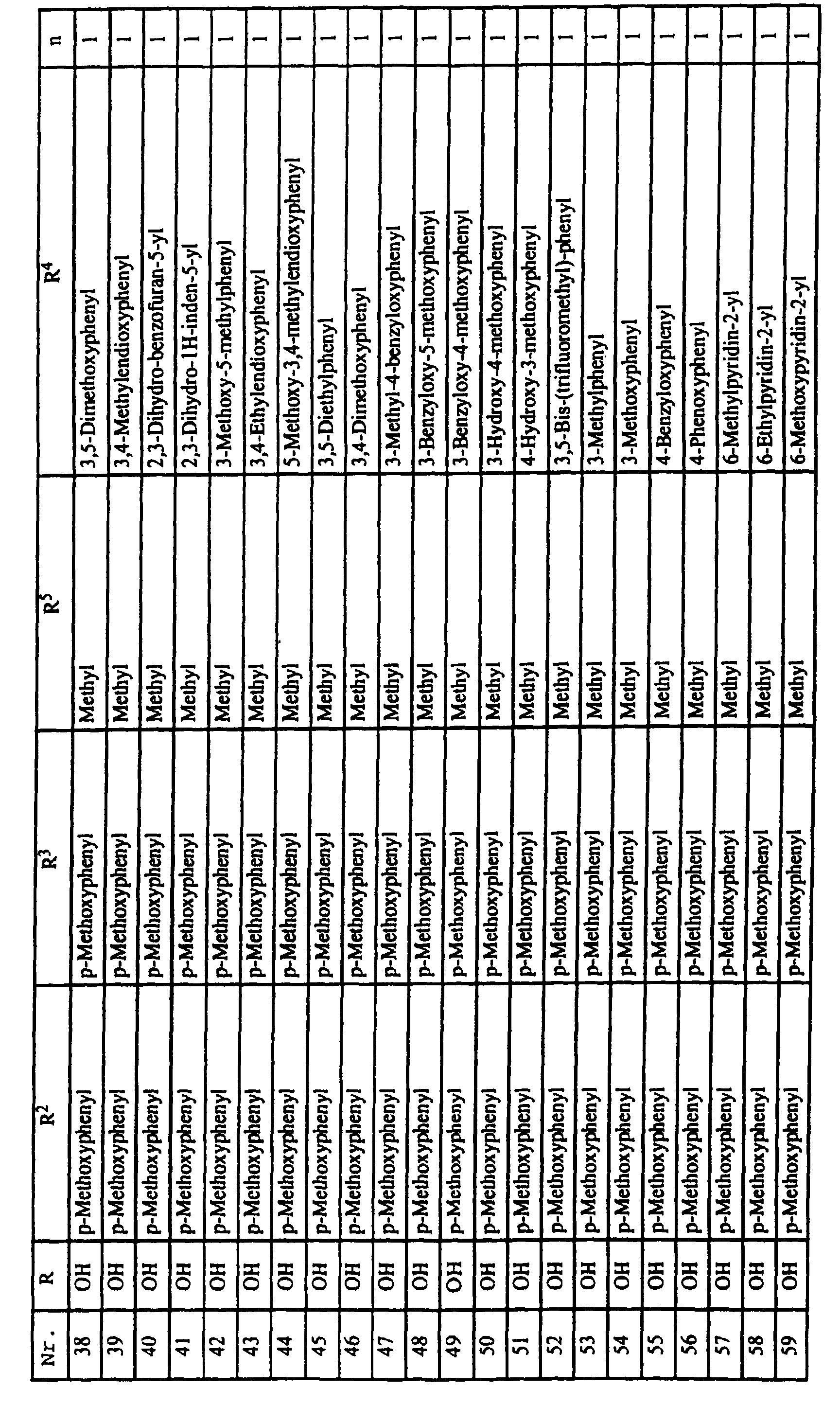

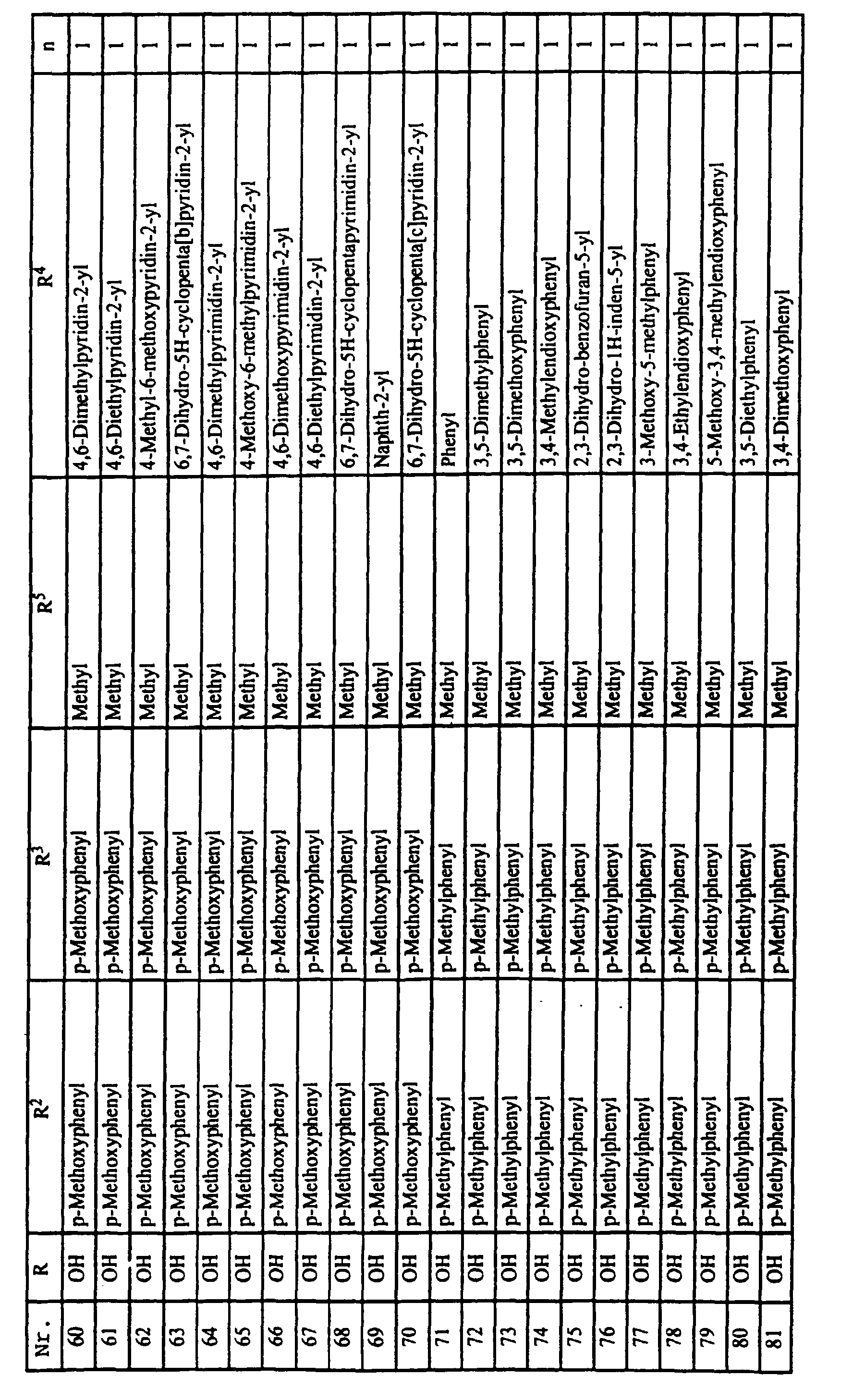

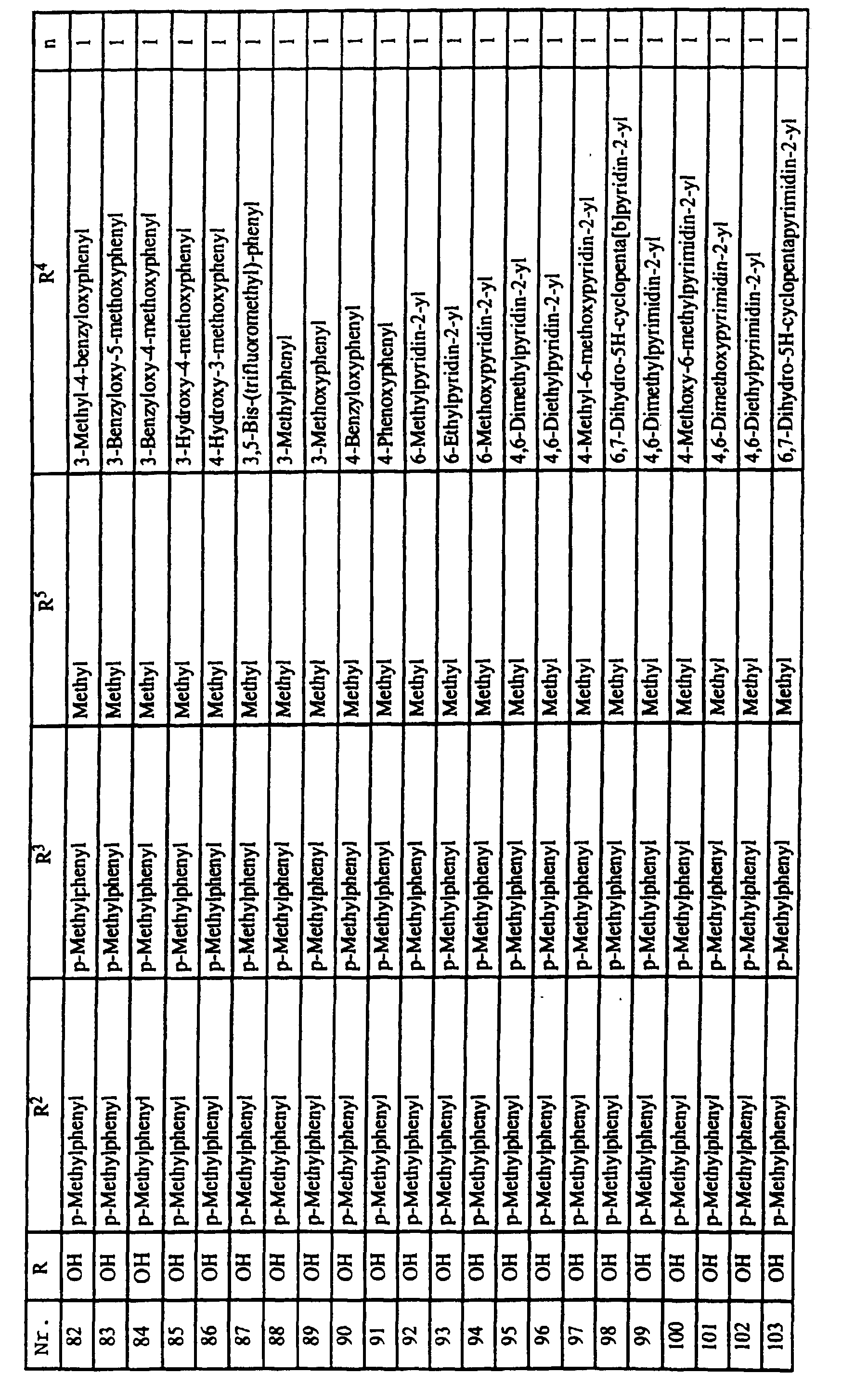

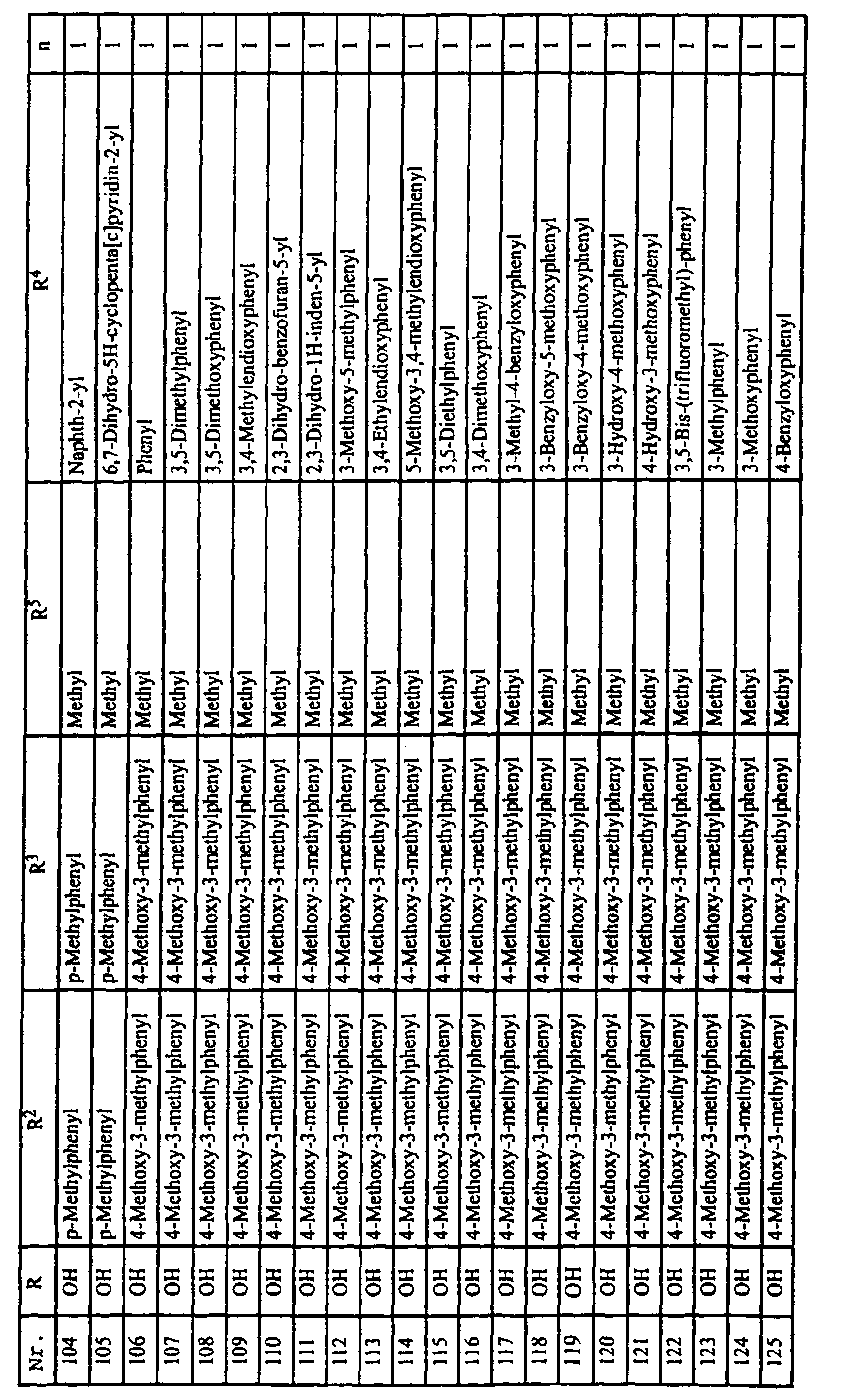

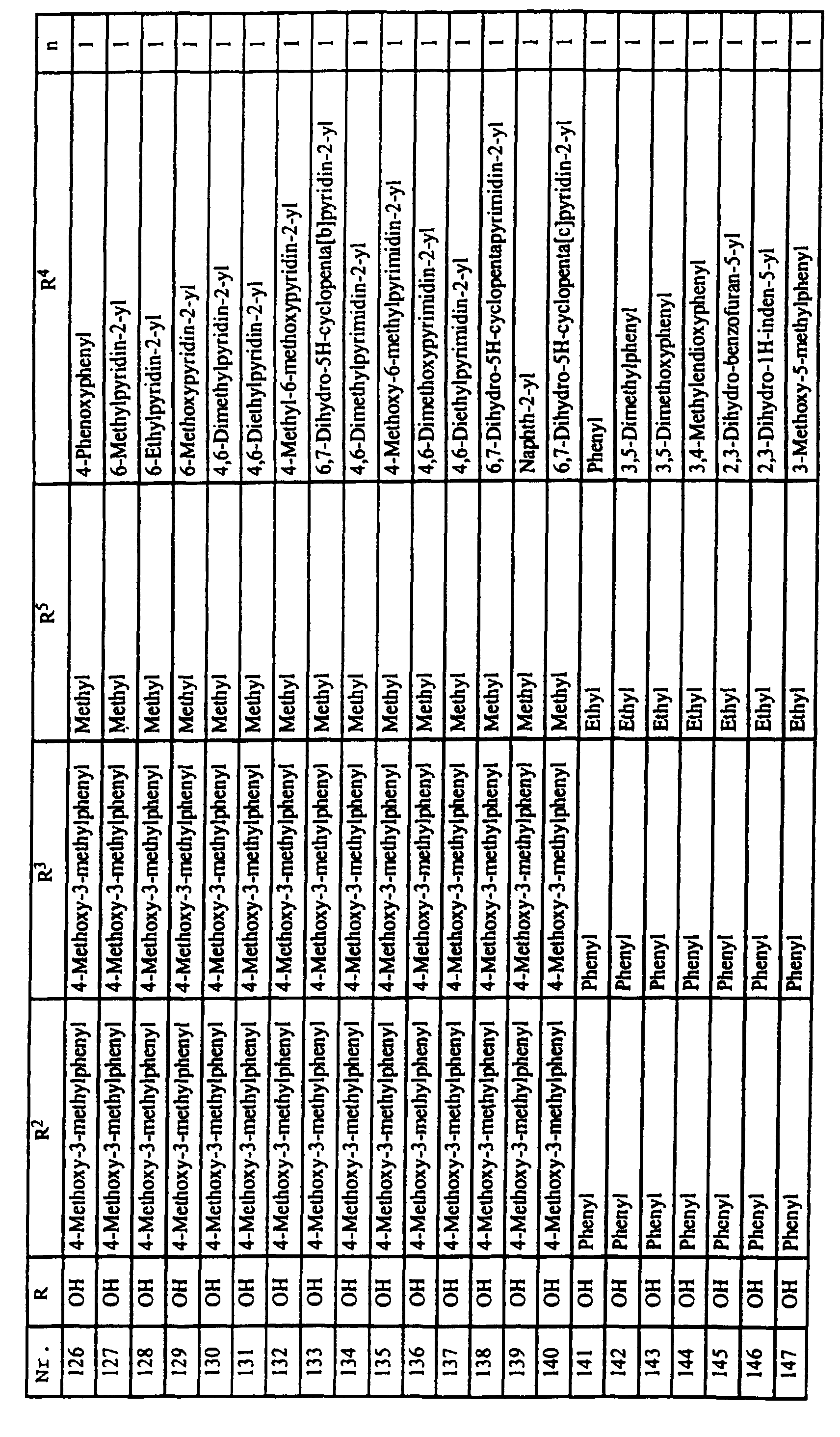

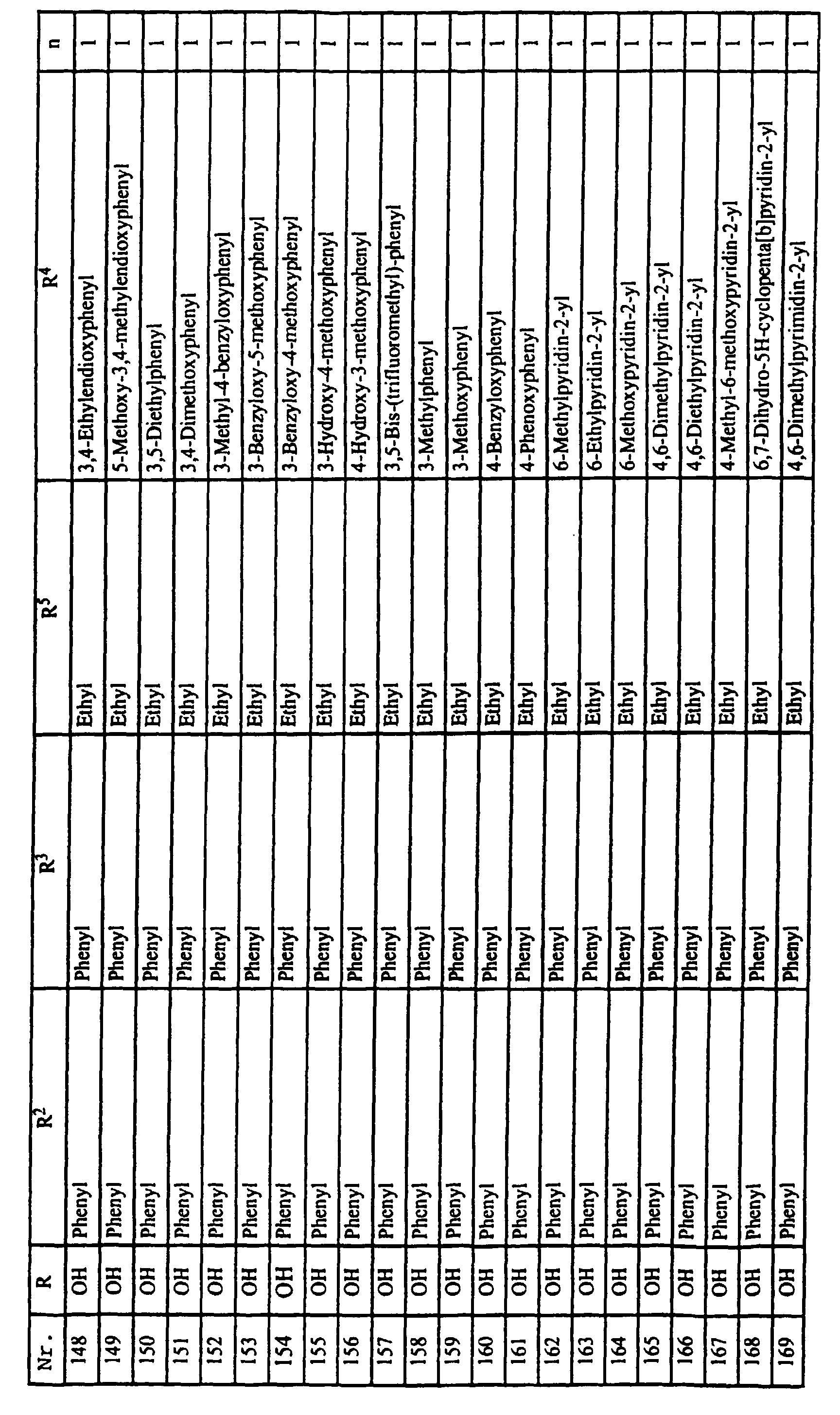

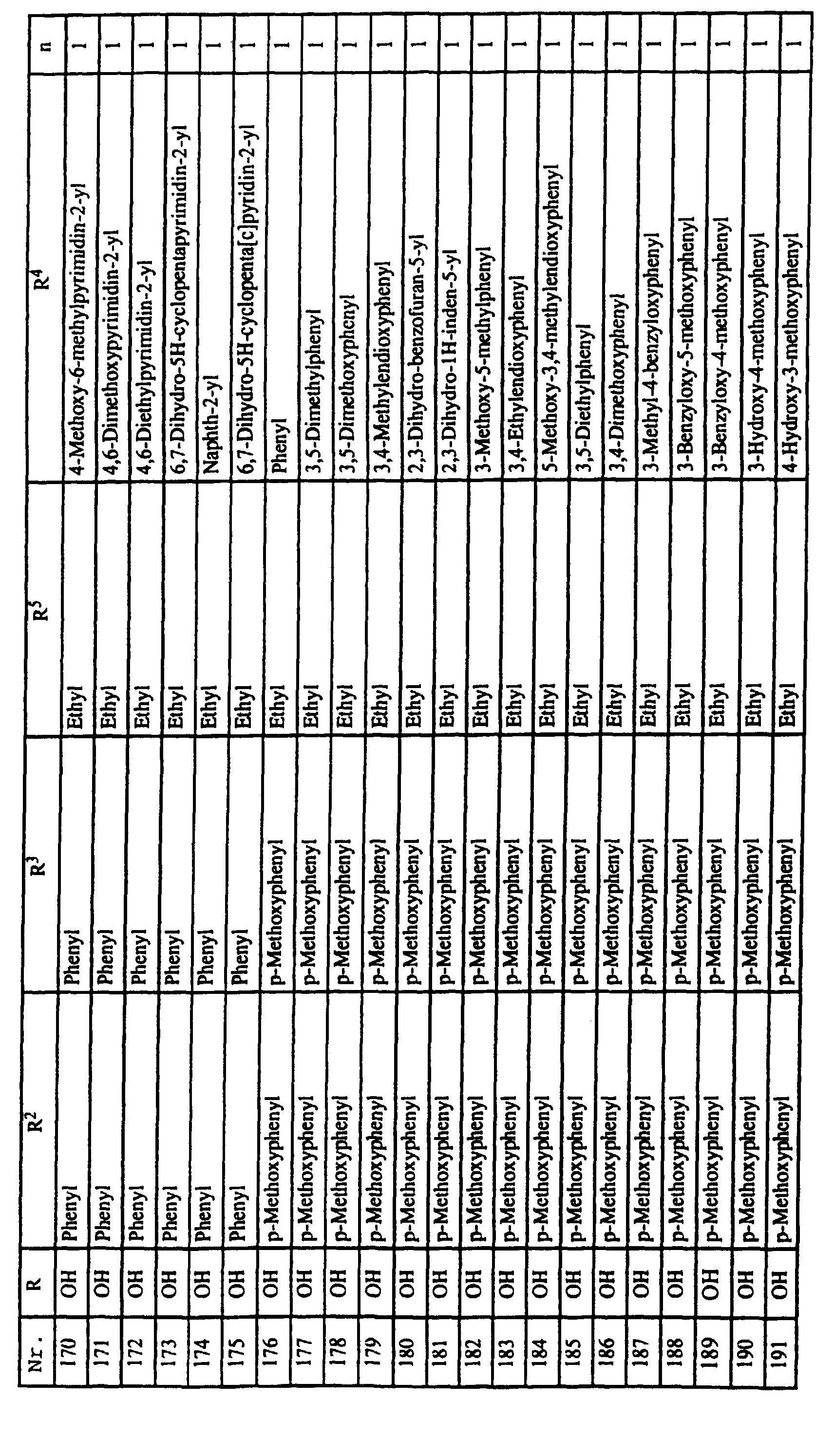

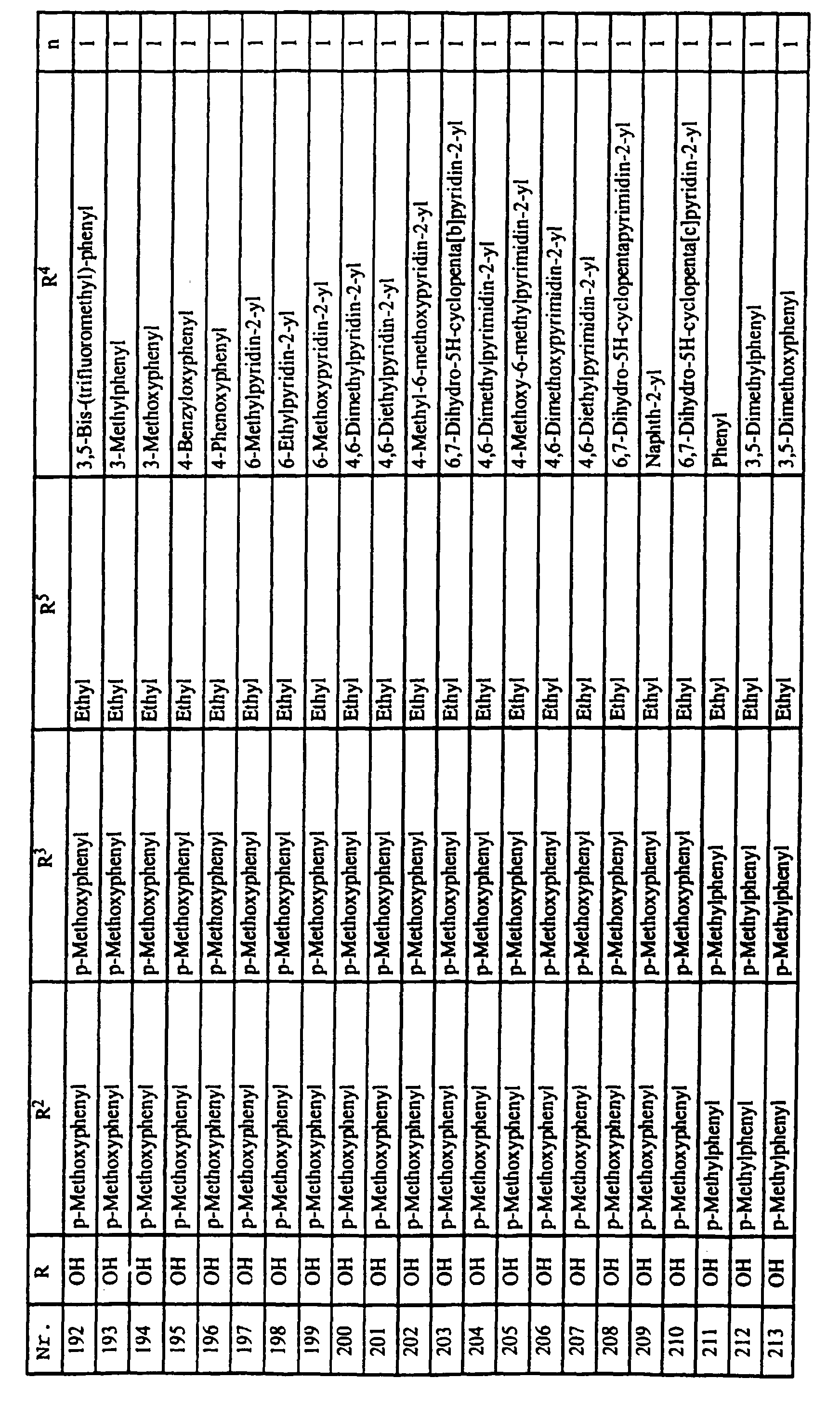

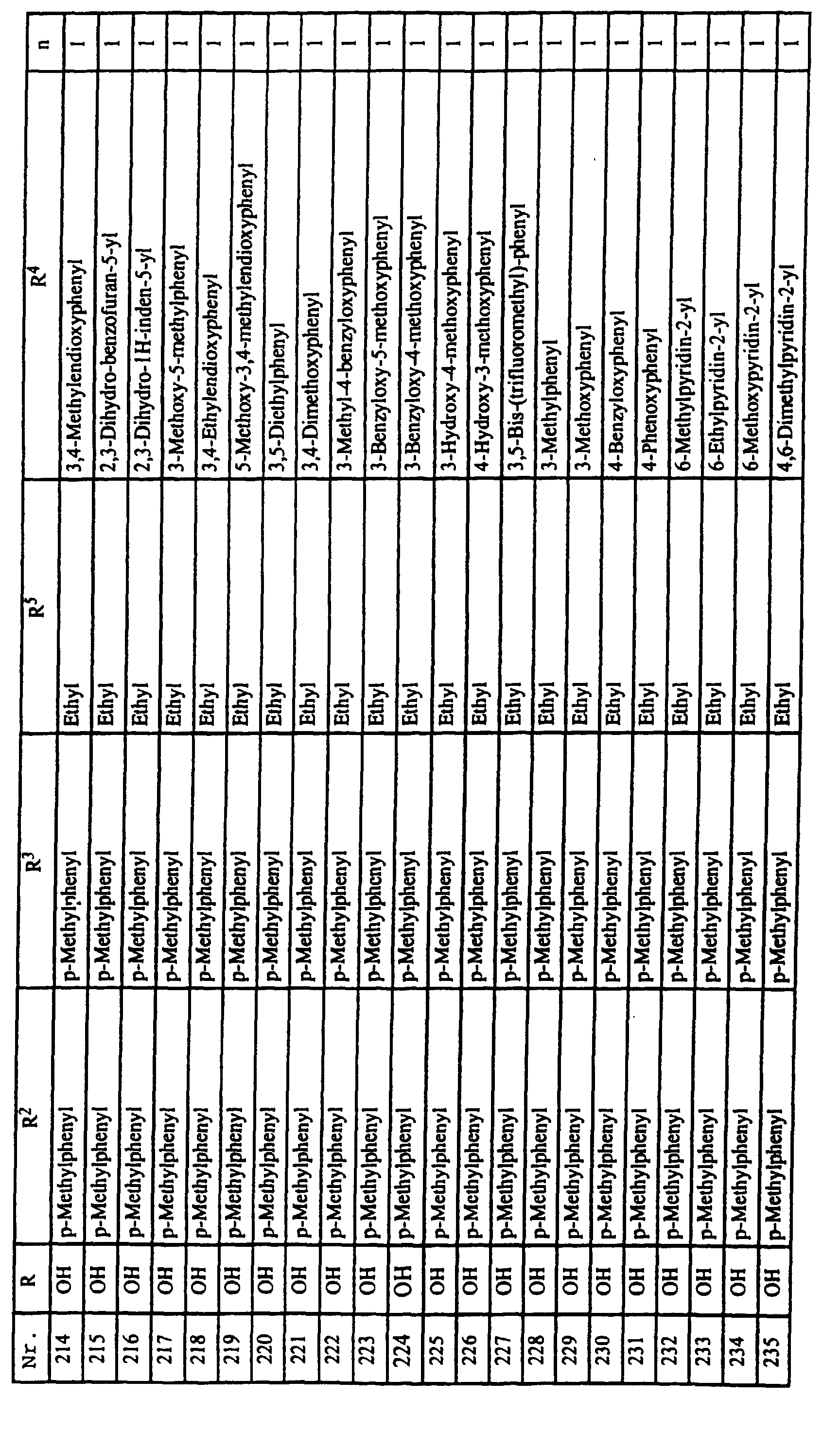

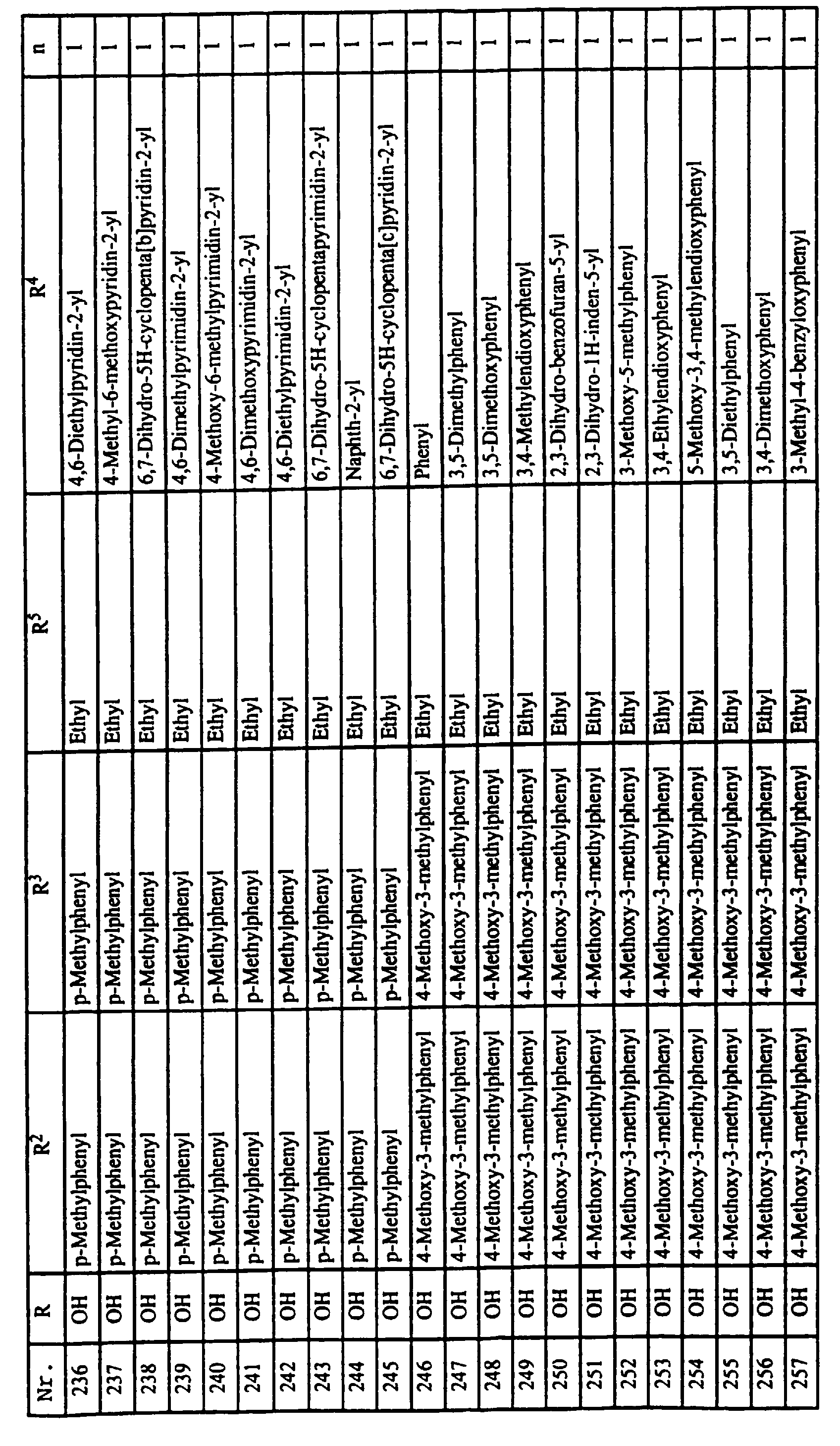

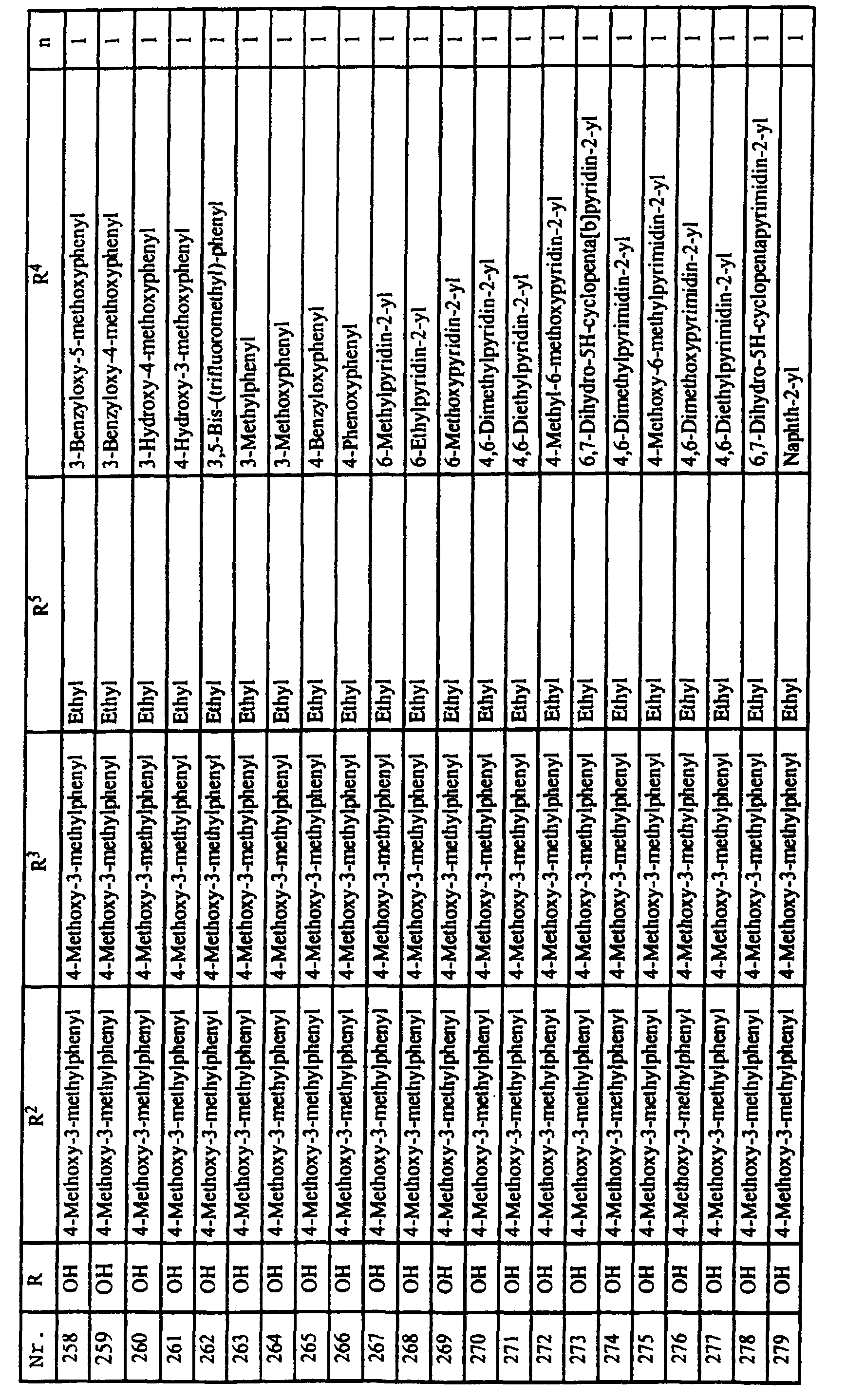

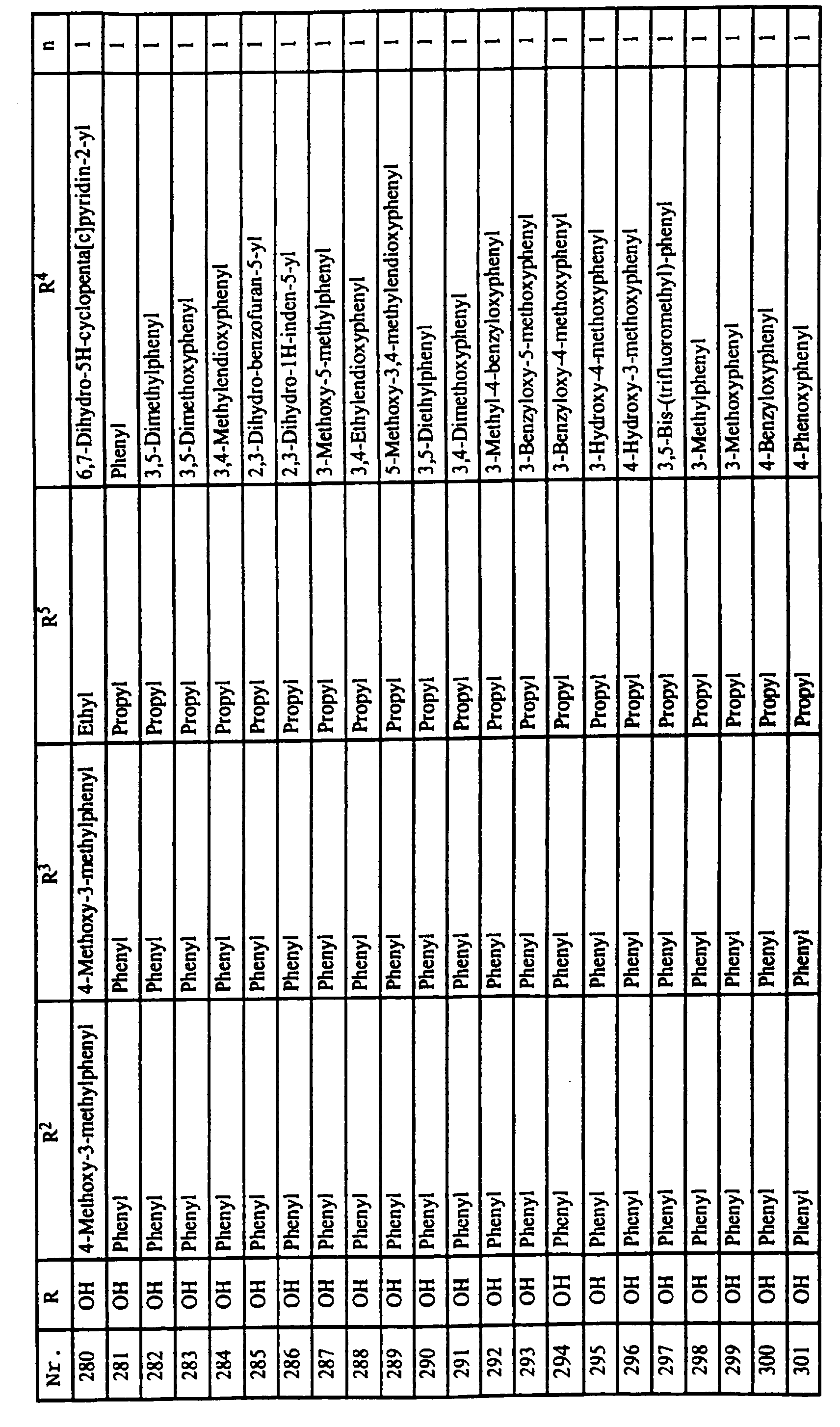

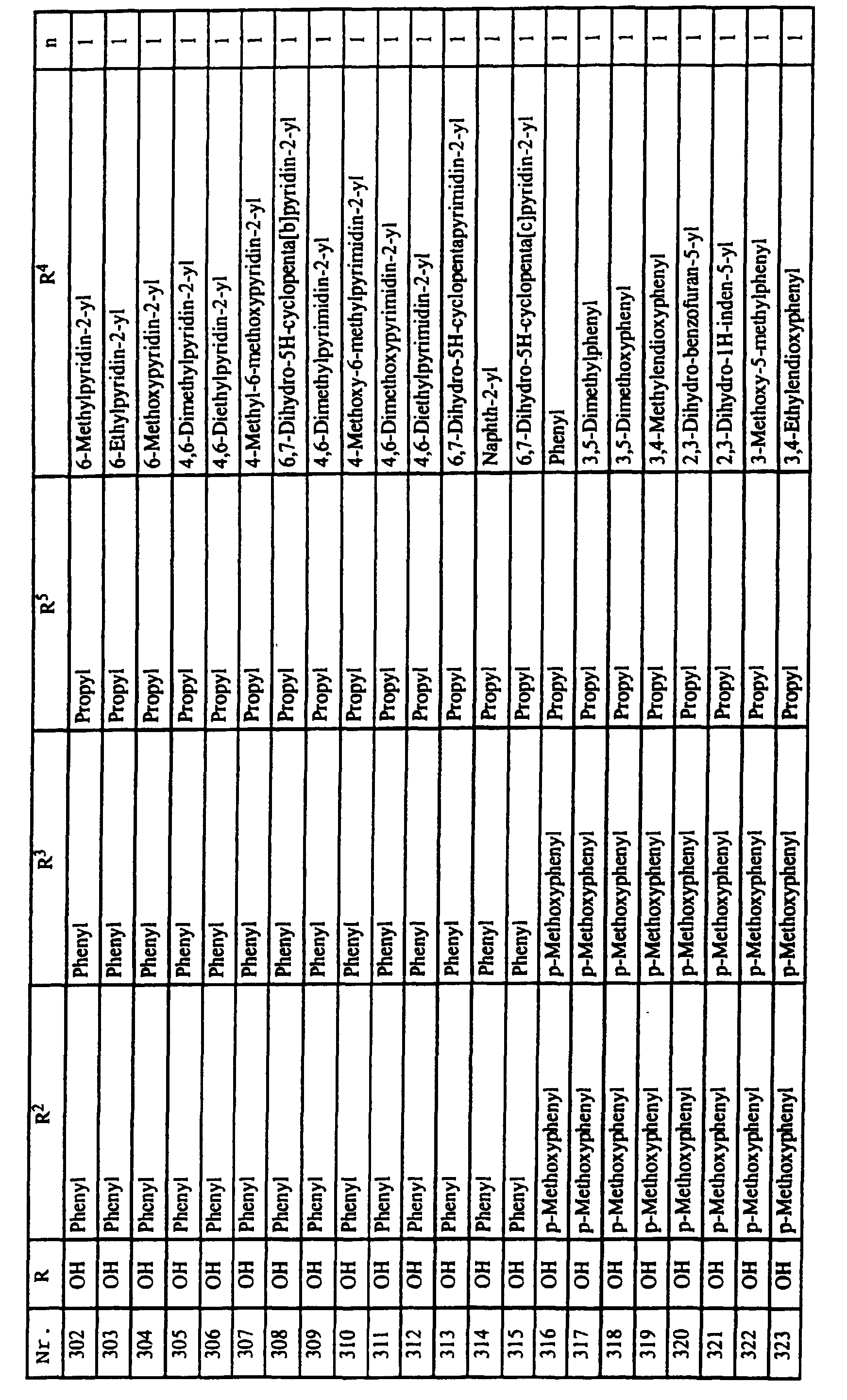

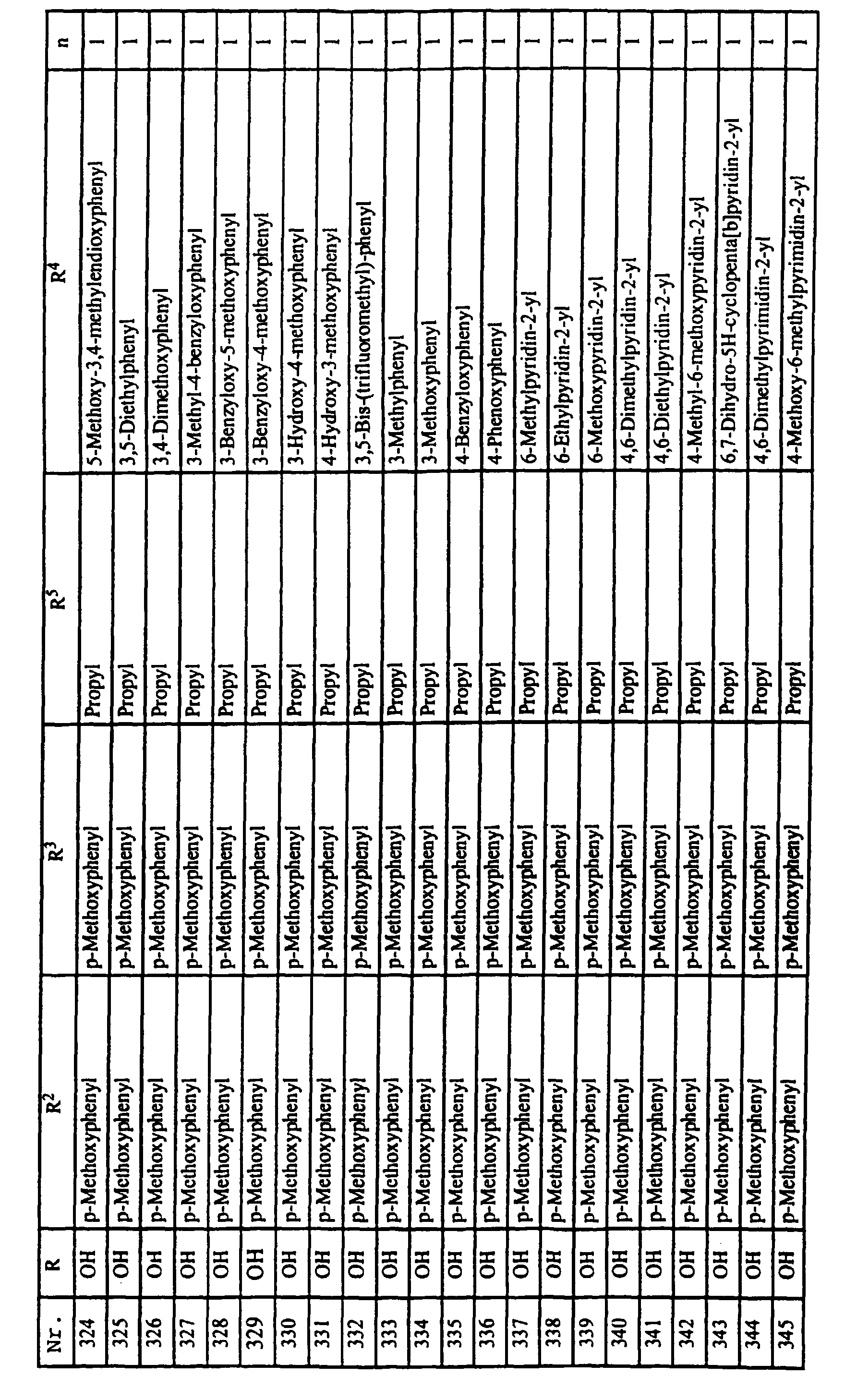

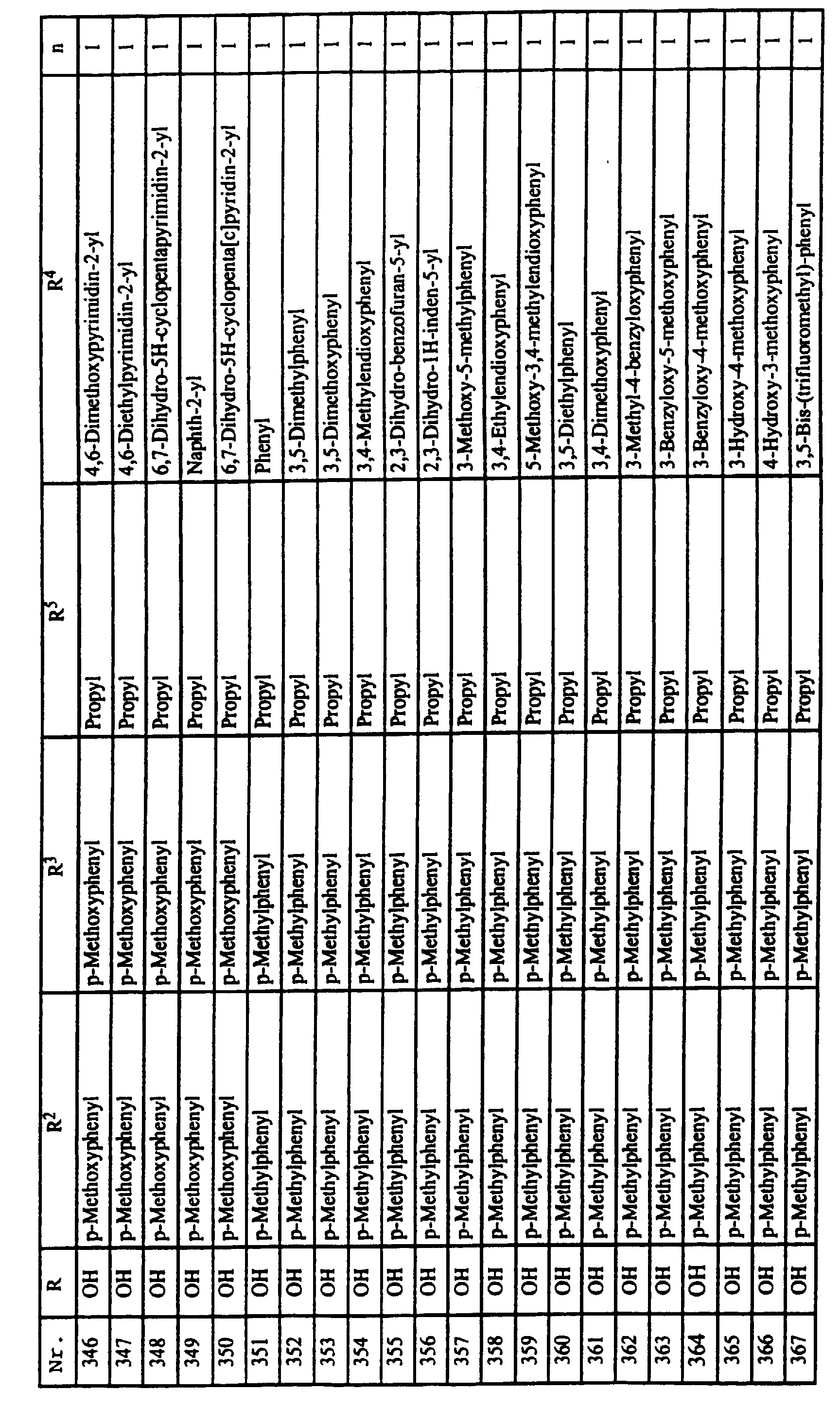

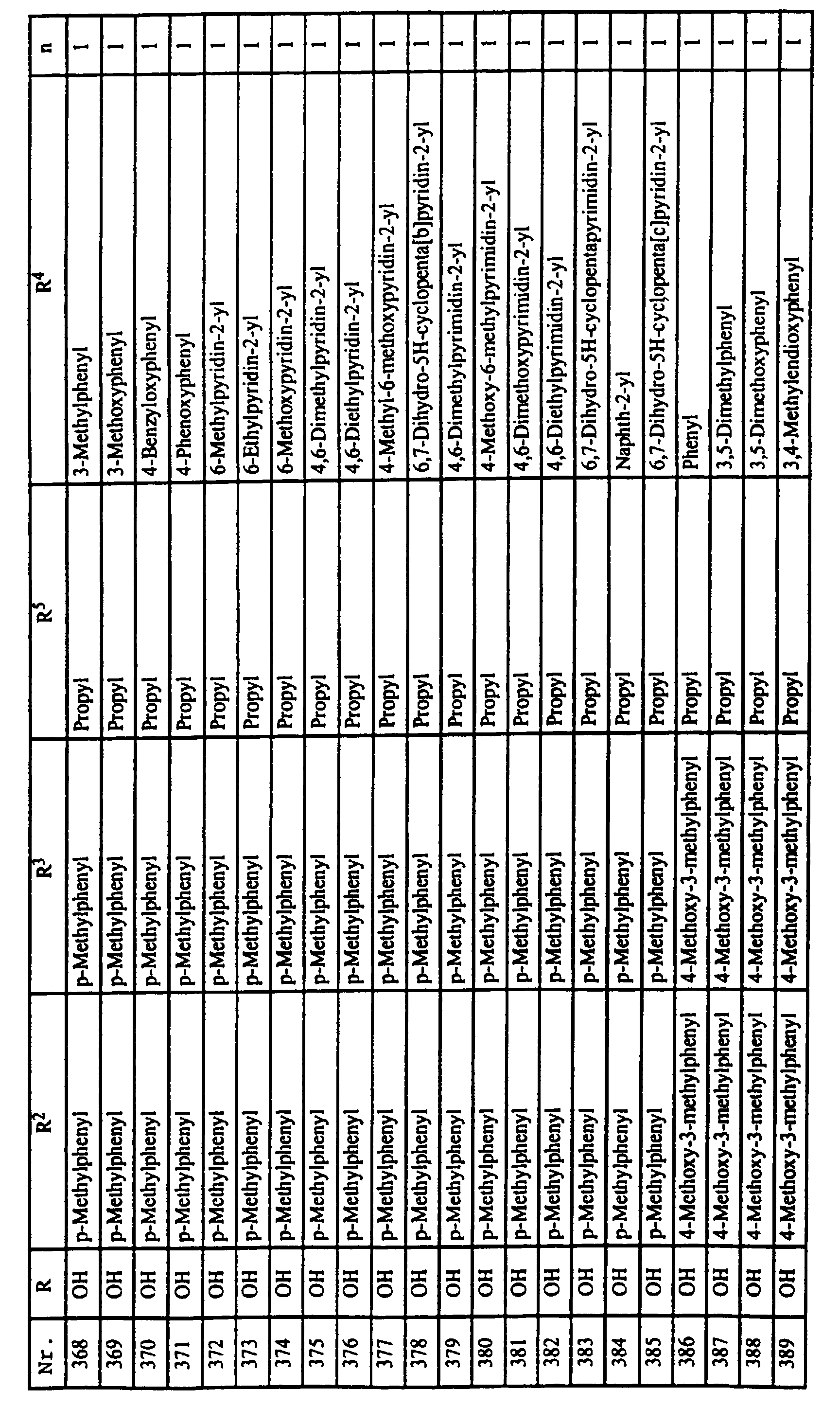

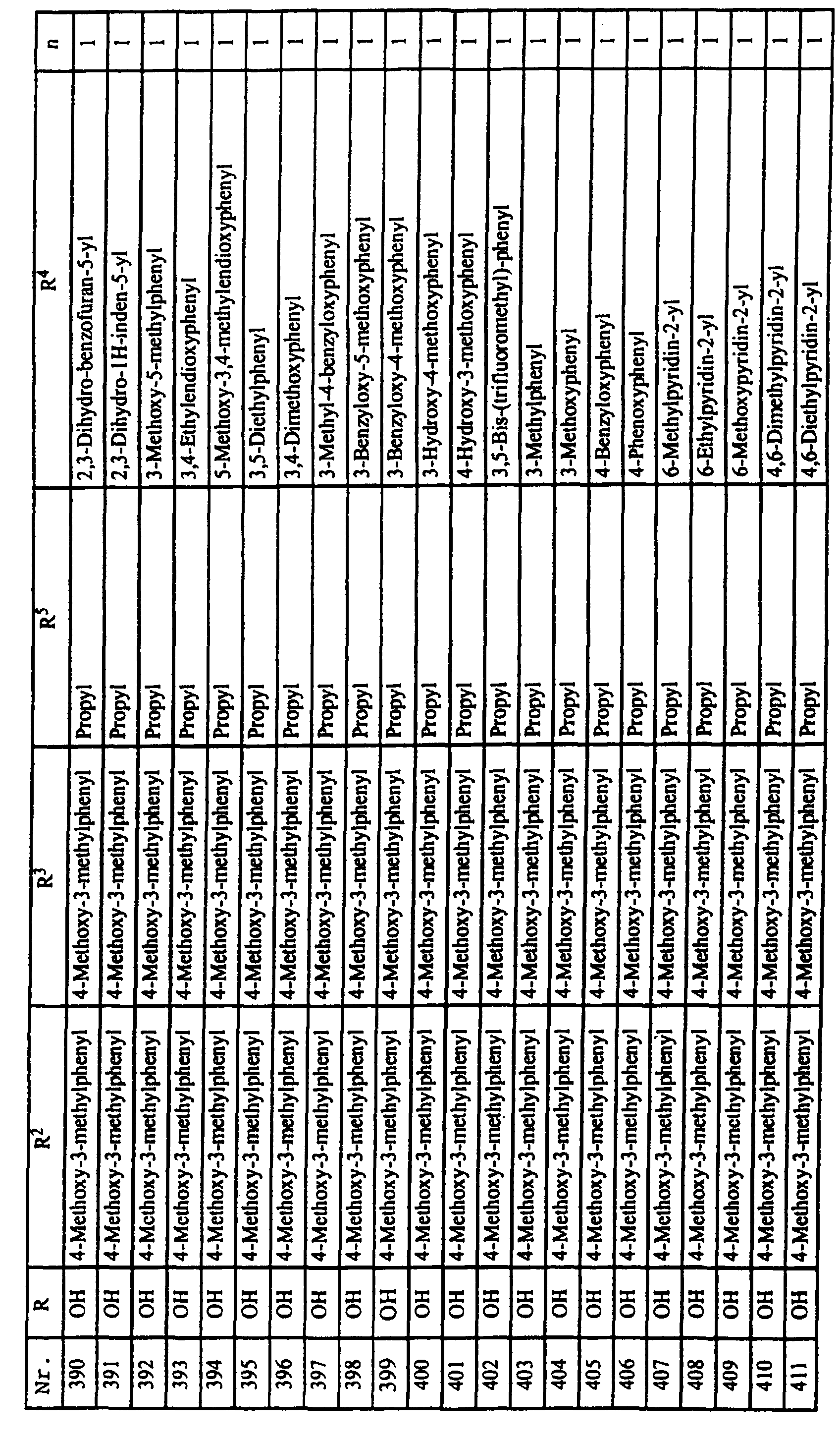

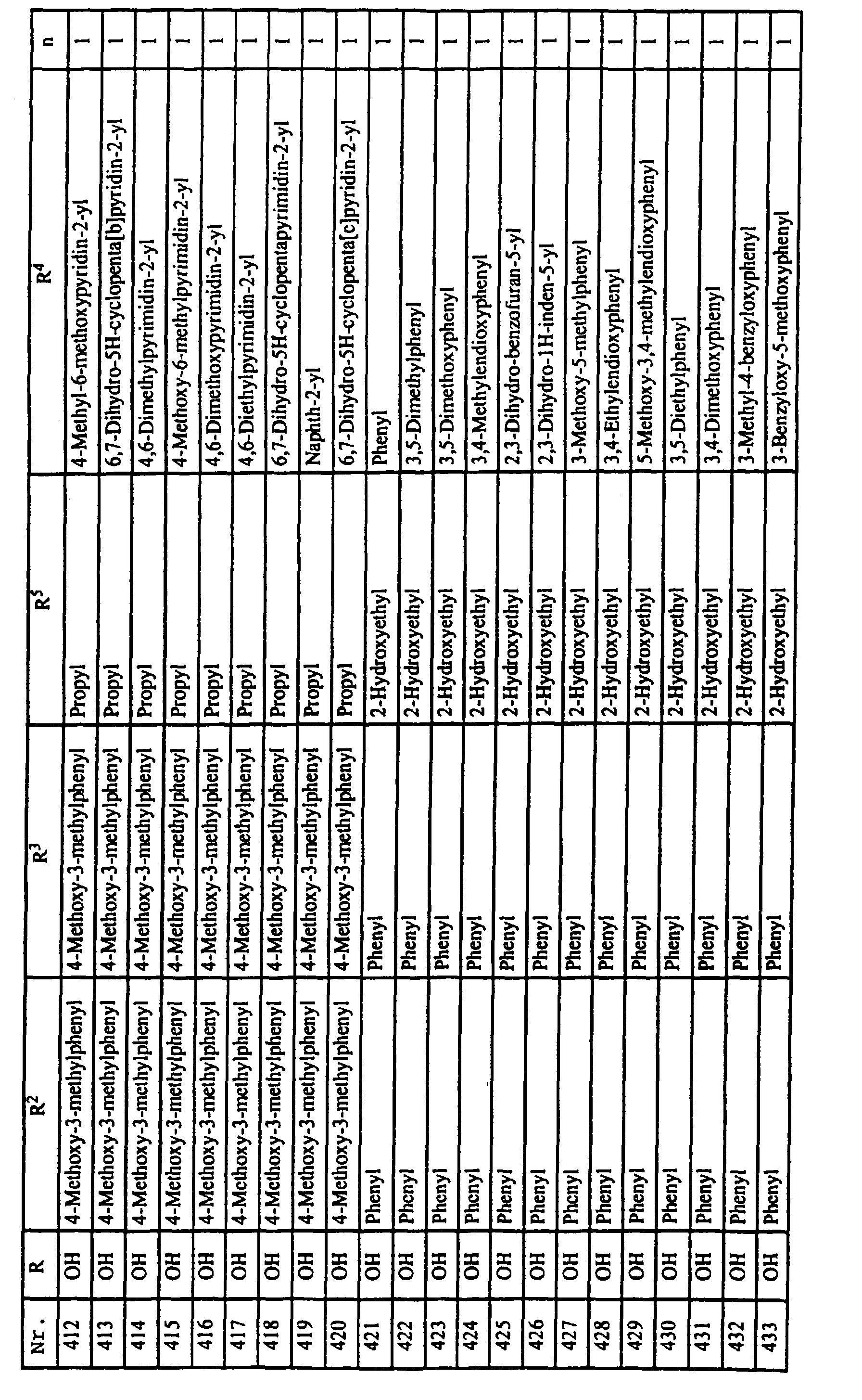

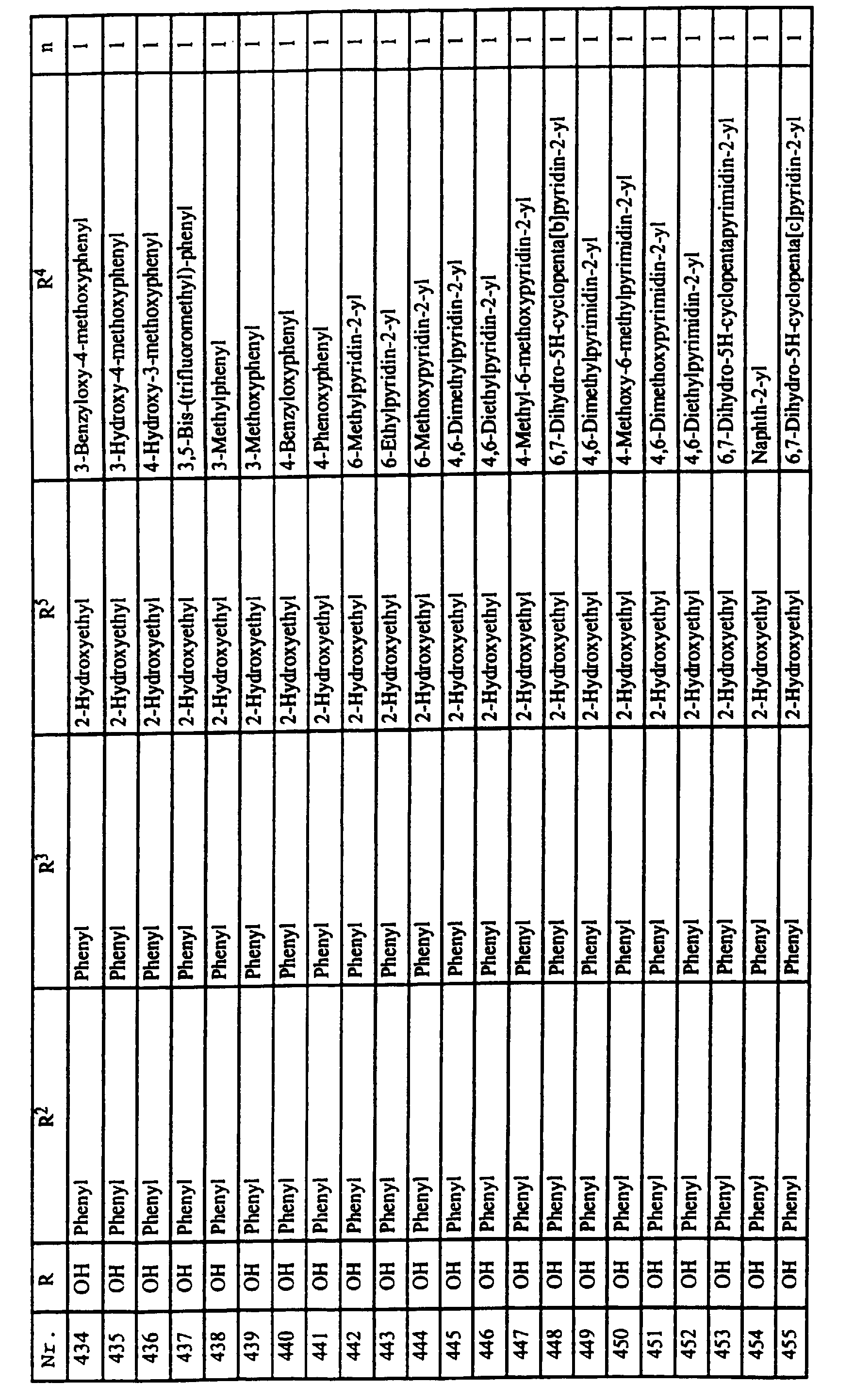

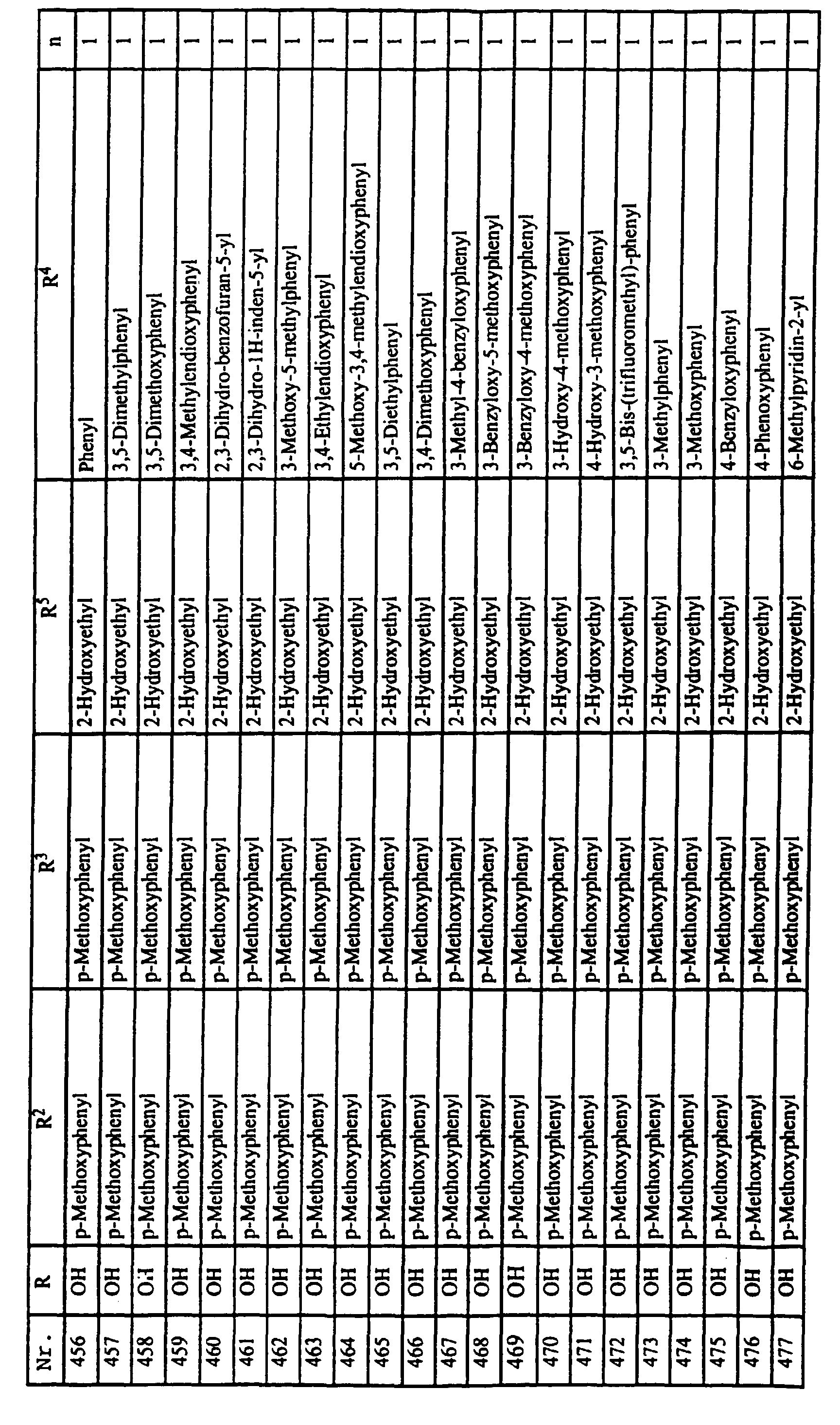

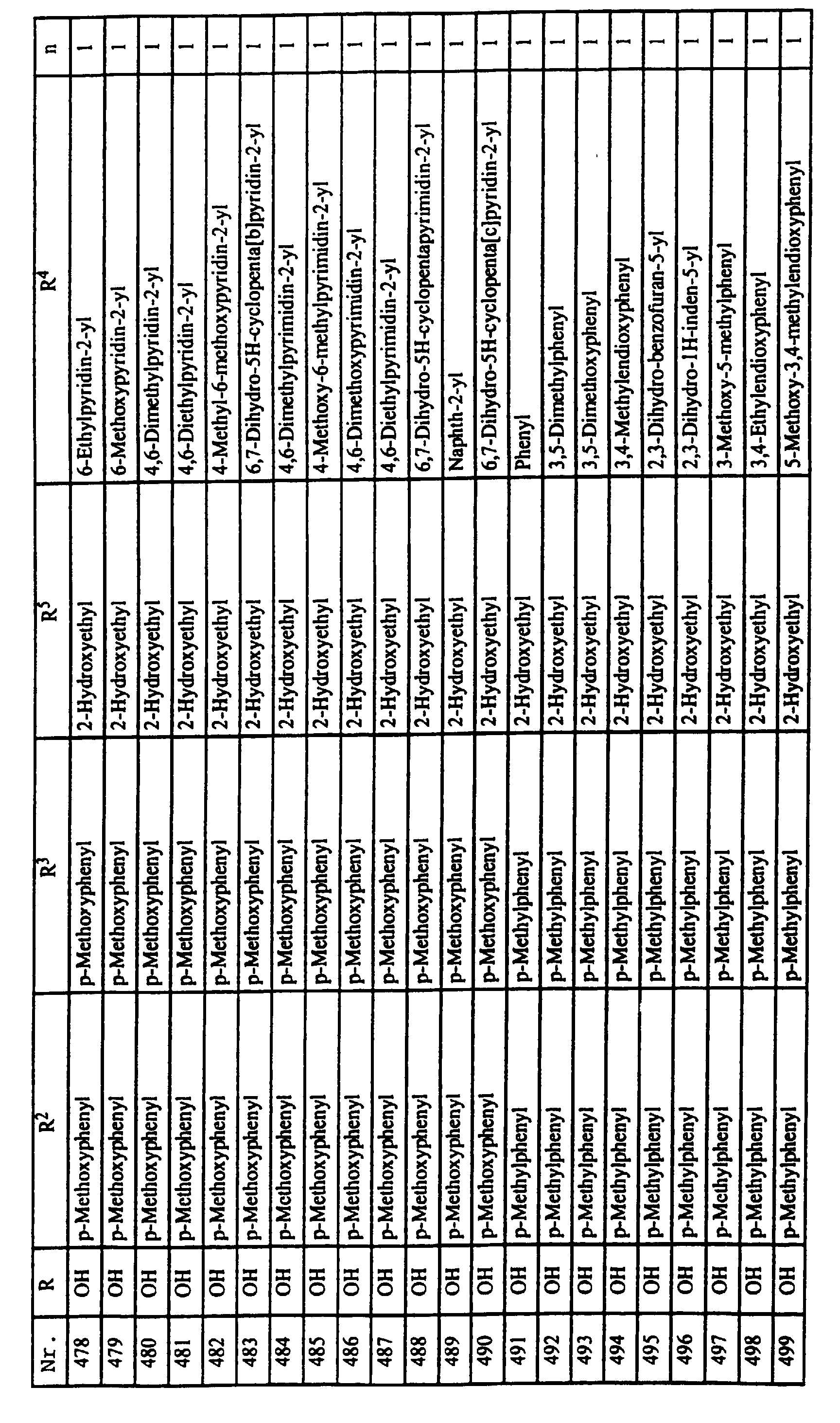

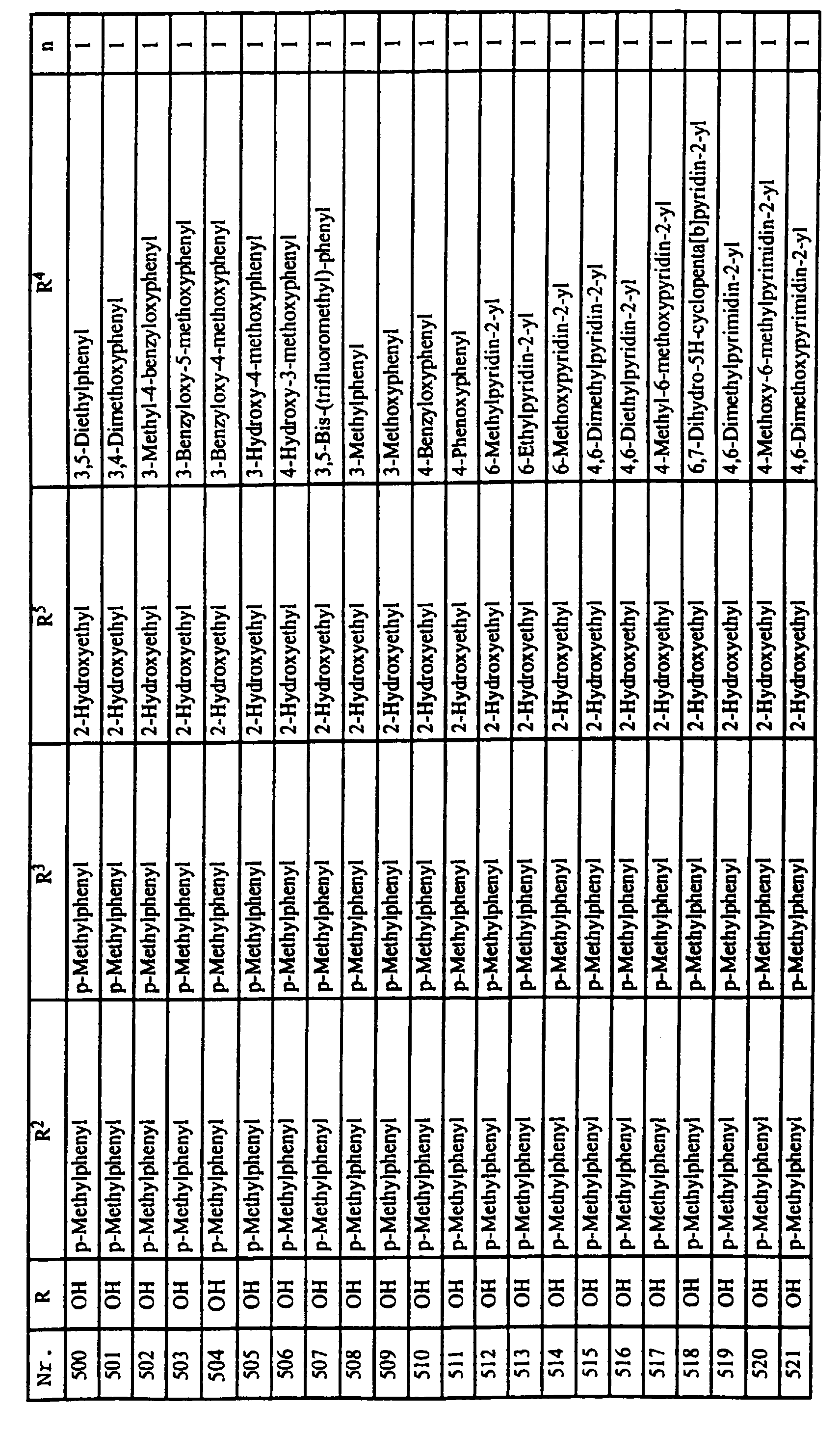

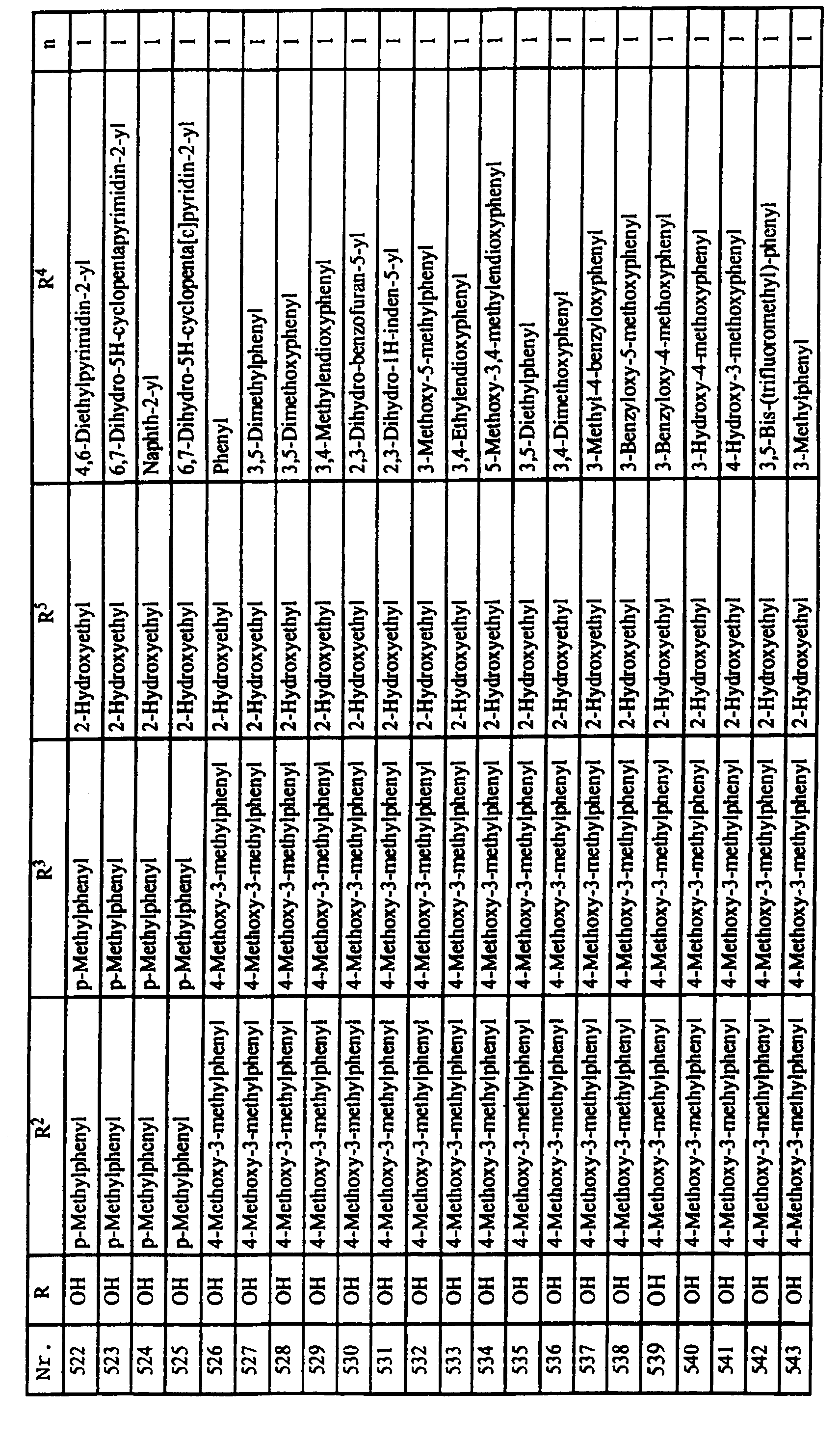

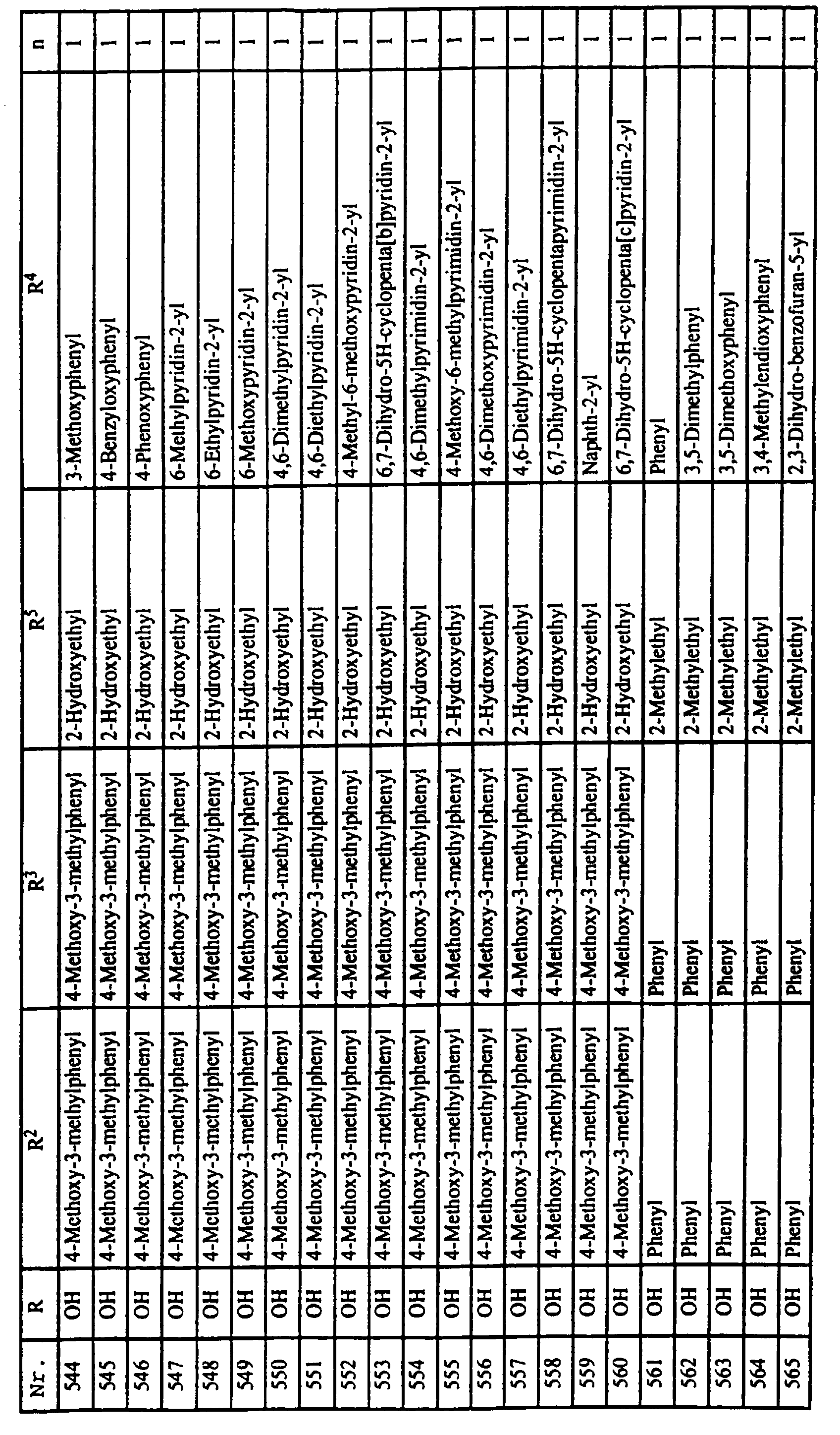

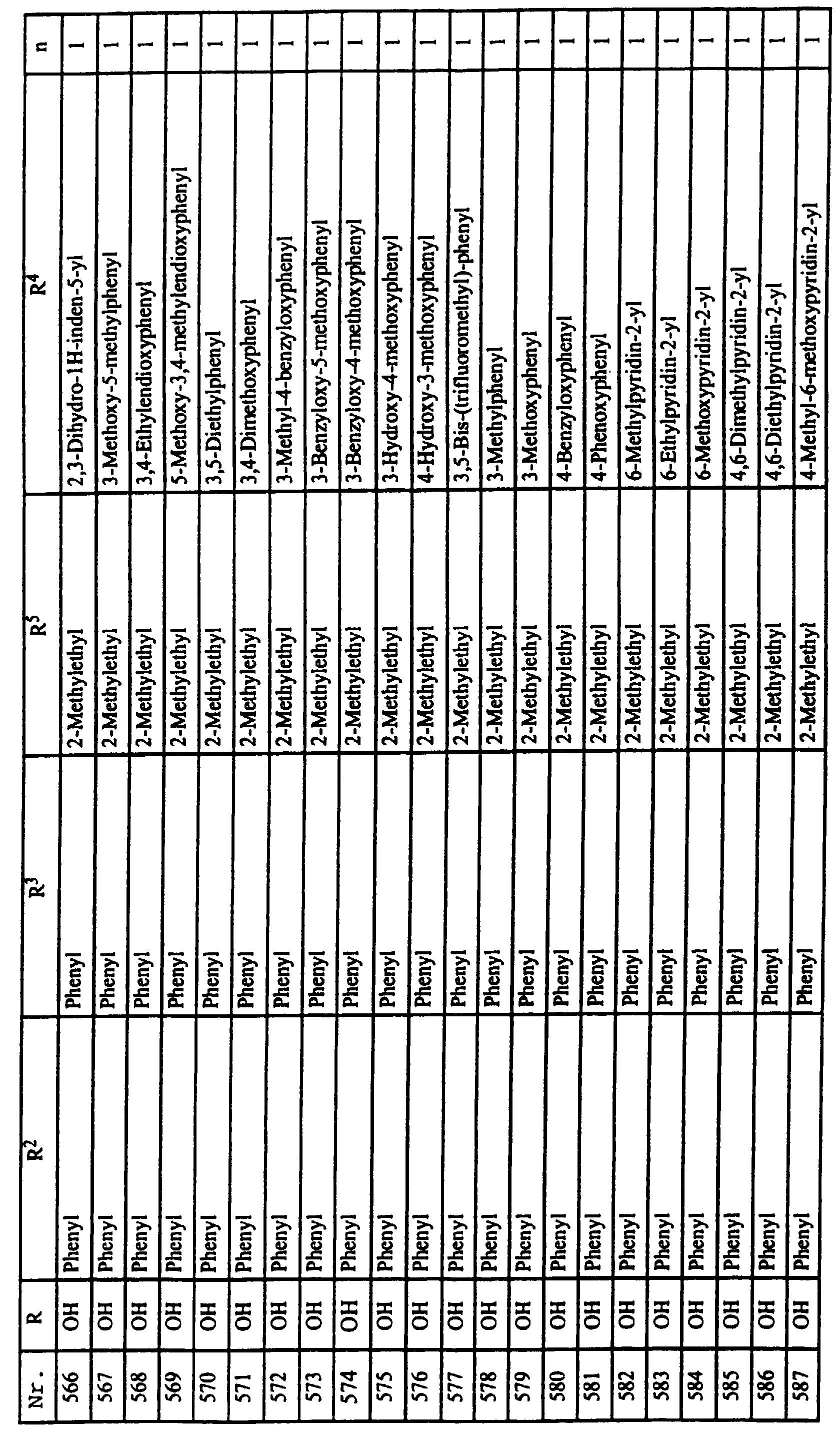

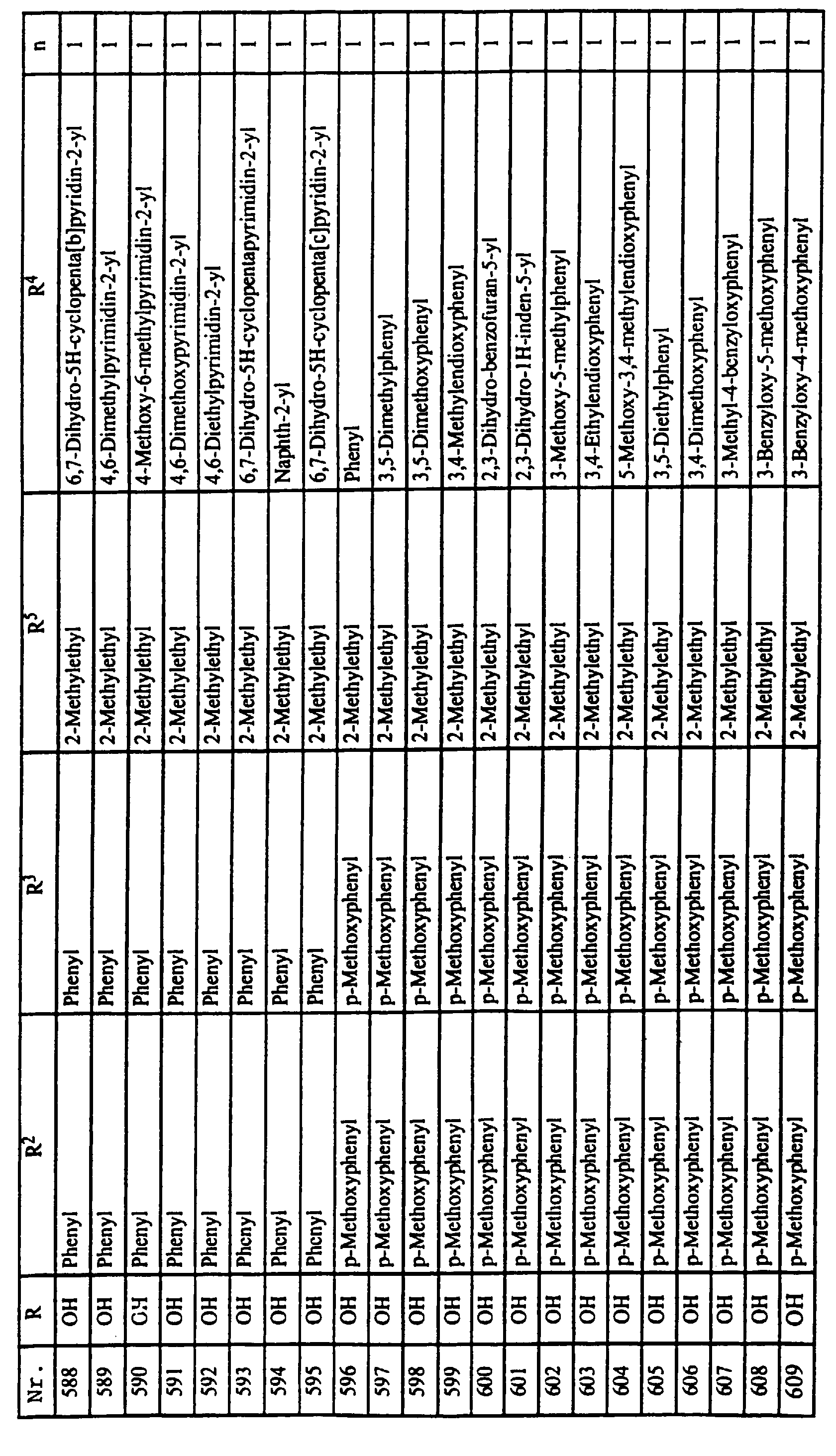

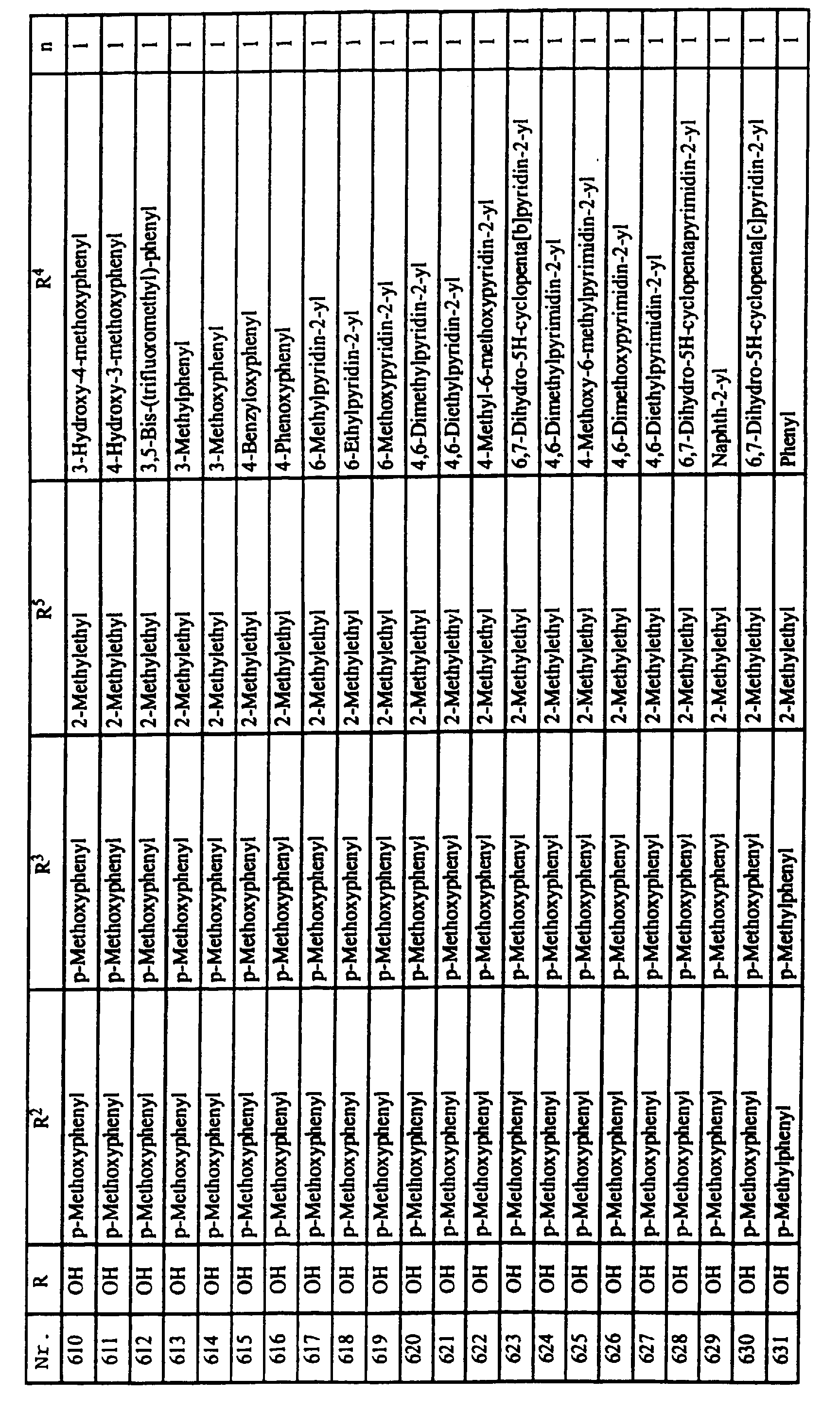

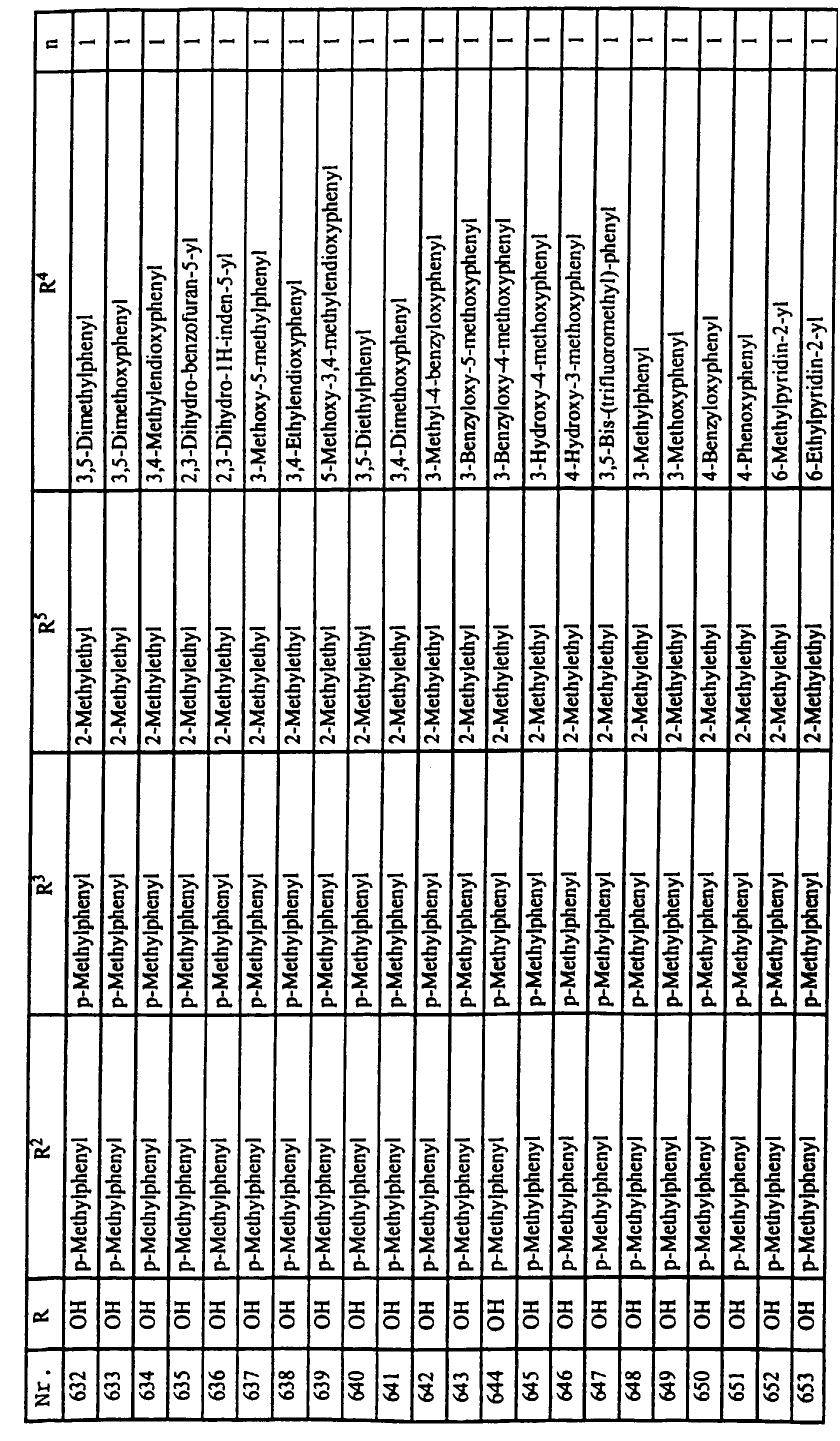

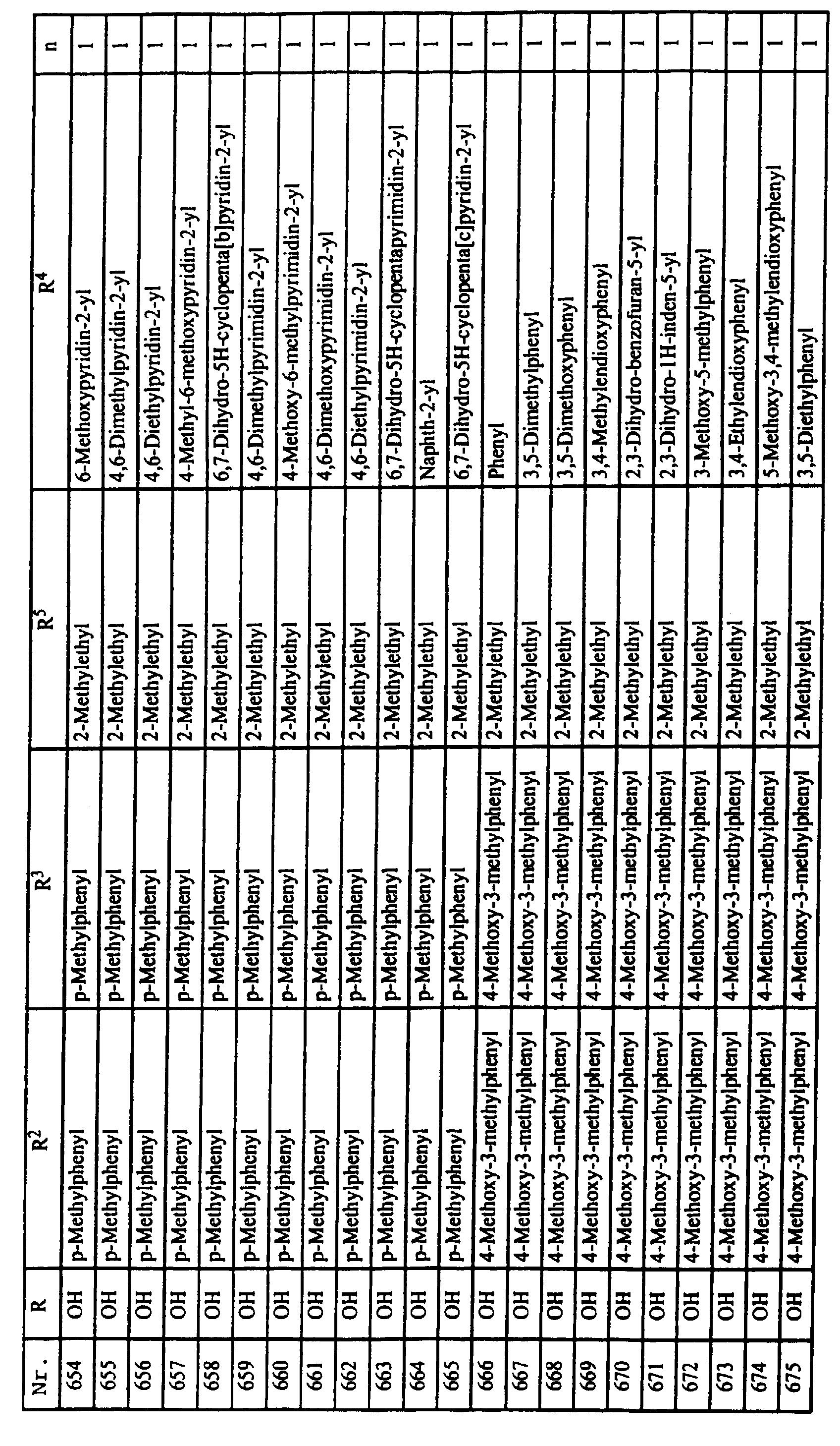

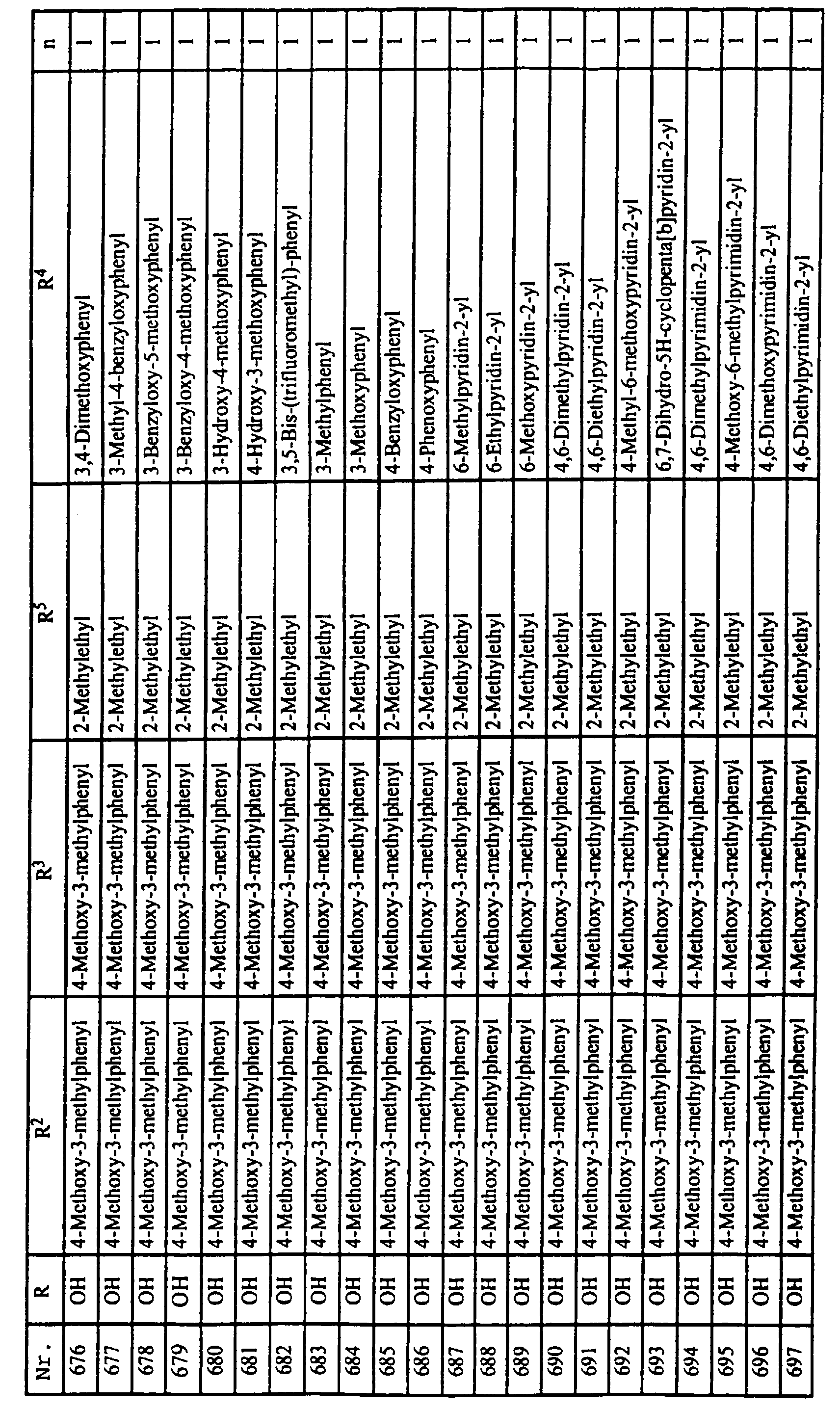

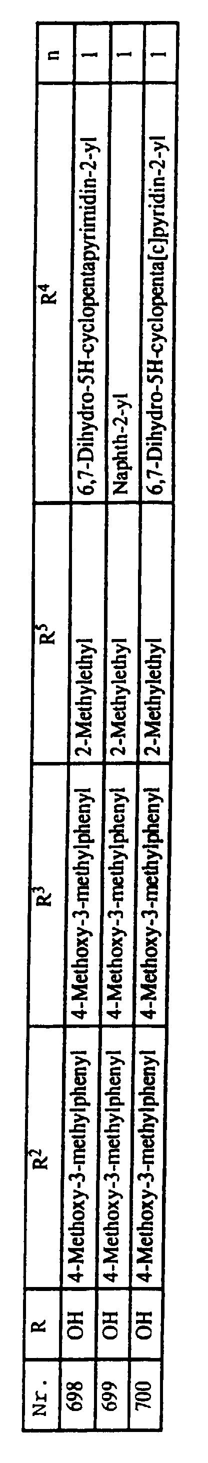

Die Verbindungen der vorliegenden Erfindung bieten ein neues therapeutisches Potential für die Behandlung von Hypertonie, pulmonalem Hochdruck, Myokardinfarkt, Angina Pectoris, akutem Nierenversagen, Niereninsuffizienz, zerebralen Vasospasmen, zerebraler Ischämie, Subarachnoidalblutungen, Migräne, Asthma, Atherosklerose, endotoxischem Schock, Endotoxin-induziertem Organversagen, intravaskulärer Koagulation, Restenose nach Angioplastie, benigne Prostata-Hyperplasie, ischämisches und durch Intoxikation verursachtes Nierenversagen bzw. Hypertonie.

Die gute Wirkung der Verbindungen läßt sich in folgenden Versuchen zeigen:

### Rezeptorbindungsstudien

Für Bindungsstudien wurden klonierte humane ET_{A}-Rezeptor-exprimierende CHO-Zellen und Meerschweinchen-Kleinhirnmembranen mit > 60 % ET_{B}- im Vergleich zu ET_{A}-Rezeptoren eingesetzt.

### Membranpräparation

Die ET_{A}-Rezeptor-exprimierenden CHO-Zellen wurden in F₁₂-Medium mit 10 % fötalem Kälberserum, 1 % Glutamin, 100 E/ml Penicillin und 0,2 % Streptomycin (Gibco BRL, Gaithersburg, MD, USA) vermehrt. Nach 48 h wurden die Zellen mit PBS gewaschen und mit 0,05 % trypsinhaltiger PBS 5 min inkubiert. Danach wurde mit F₁₂-Medium neutralisiert und die Zellen durch Zentrifugation bei 300 x g gesammelt. Zur Lyse der Zellen wurde kurz das Pellet mit Lysispuffer (5 mM Tris-HCl, pH 7,4 mit 10 % Glycerin) gewaschen und danach in einer Konzentration von 10⁷-Zellen/ml Lysispuffer 30 min bei 4°C inkubiert. Die Membranen wurden bei 20.000 x g 10 min zentrifugiert und das Pellet in flüssigem Stickstoff gelagert.

Meerschweinchenkleinhirne wurden im Potter-Elvejhem-Homogenisator homogenisiert und durch differentielle Zentrifugation 10 min bei 1.000 x g und wiederholte Zentrifugation des Überstandes 10 min bei 20.000 x g gewonnen.

### Bindungstests

Für den ET_{A}- und ET_{B}-Rezeptorbindungstest wurden die Membranen in Inkubationspuffer (50 mM Tris-HCl, pH 7,4 mit 5 mM MnCl₂, 40 µg/ml Bacitracin und 0,2 % BSA) in einer Konzentration von 50 µg Protein pro Testansatz suspendiert und bei 25°C mit 25 pM ¹²⁵J-ET₁ (ET_{A}-Rezeptortest) oder 25 pM ¹²⁵J-RZ₃ (ET_{B}-Rezeptortest) in Anwesenheit und Abwesenheit von Testsubstanz inkubiert. Die unspezifische Bindung wurde mit 10⁻⁷ M ET₁ bestimmt. Nach 30 min wurde der freie und der gebundene Radioligand durch Filtration über GF/B Glasfaserfilter (Whatman, England) an einem Skatron-Zellsammler (Skatron, Lier, Norwegen) getrennt und die Filter mit eiskaltem Tris-HCl-Puffer, pH 7,4 mit 0,2 % BSA gewaschen. Die auf den Filtern gesammelte Radioaktivität wurde mit einem Packard 2200 CA Flüssigkeitszintillationszähler quantifiziert.

Funktionelles in vitro-Testsystem für die Suche nach Endothelinrezeptor (Subtyp A)-Antagonisten

Dieses Testsystem ist ein funktioneller, auf Zellen basierender Test für Endothelinrezeptoren. Bestimmte Zellen zeigen, wenn sie mit Endothelin 1 (ET1) stimuliert werden, einen Anstieg der intrazellulären Calciumkonzentration. Dieser Anstieg kann in intakten Zellen, die mit Calcium-sensitiven Farbstoffen beladen wurden, gemessen werden.

Aus Ratten isolierte 1-Fibroblasten, bei denen ein endogener Endothelinrezeptor vom A-Subtyp nachgewiesen wurde, wurden mit dem Fluoreszenzfarbstoff Fura 2-an wie folgt beladen: Nach Trypsinierung wurden die Zellen in Puffer A (120 mM NaCl, 5 mM KCl, 1,5 mM MgCl₂, 1 mM CaCl₂, 25 mM HEPES, 10 mM Glucose, pH 7,4) bis zu einer Dichte von 2 x 10⁶/ml resuspendiert und in 30 min bei 37°C im Dunkeln mit Fura 2-am (2 µM), Pluronic F-127 (0,04 %) und DMSO (0,2 %) inkubiert. Danach wurden die Zellen zweimal mit Puffer A gewaschen und zu 2 x 10⁶/ml resuspendiert.

Das Fluoreszenzsignal von 2 x 10⁵ Zellen pro ml bei Ex/Em 380/510 wurde bei 30°C kontinuierlich registriert. Zu den Zellen wurden die Testsubstanzen und nach einer Inkubationszeit von 3 min ET1 zugegeben. Über einen Zeitraum von 30 min wurde die maximale Änderung der Fluoreszenz bestimmt. Die Antwort der Zellen auf ET1 ohne vorherige Zugabe einer Testsubstanz diente als Kontrolle und wurde gleich 100 % gesetzt.

### Testung der ET-Antagonisten in vivo

Männliche 250 - 300 g schwere SD-Ratten wurden mit Amobarbital narkotisiert, künstlich beatmet, vagotomisiert und despinalisiert. Die Arteria carotis und Vena jugularis wurden katheterisiert.

In Kontrolltieren führt die intravenöse Gabe von 1 µg/kg ET1 zu einem deutlichen Blutdruckanstieg, der über einen längeren Zeitraum anhält.

Den Testtieren wurde 5 bzw. 30 min. vor der ET1 Gabe die Testverbindungen i.v. injiziert (1 ml/kg). Zur Bestimmung der ET-antagonistischen Eigenschaften wurde der Blutdruckanstieg in den Testtieren mit dem in den Kontrolltieren verglichen.

### Endothelin-1 induzierter "sudden death" an Mäusen

Das Testprinzip besteht in der Hemmung des durch Endothelin verursachten plötzlichen Herztodes der Maus, der wahrscheinlich durch Verengung der Herzkranzgefäße bedingt ist, durch Vorbehandlung mit Endothelin-Rezeptorantagonisten. Nach intravenöser Injektion von 10 nmol/kg Endothelin im Volumen von 5 ml/kg Körpergewicht kommt es innerhalb weniger Minuten zum Tod der Tiere.

Die letale Endothelin-1 Dosis wird jeweils an einem kleinen Tierkollektiv überprüft. Wird die Prüfsubstanz intravenös appliziert, erfolgt meist 5 min danach die im Referenzkollektiv letale Endothelin-1 Injektion. Bei anderen Applikationsarten verlängern sich die Vorgabezeiten, gegebenenfalls bis zu mehreren Stunden.

Die Überlebensrate wird dokumentiert und effektive Dosen, die 50 % der Tiere 24 h oder länger gegen den Endothelin-Herztod schützen (ED 50) werden ermittelt.

### Funktioneller Gefäßtest für Endothelin-Rezeptorantagonisten

An Aortensegmenten des Kaninchens wird nach einer Vorspannung von 2 g und einer Relaxationszeit von 1 h in Krebs-Henseleitlösung bei 37°C und einem pH-Wert zwischen 7,3 und 7,4 zunächst eine K⁺-Kontraktur ausgelöst. Nach Auswaschen wird eine Endothelin-Dosiswirkungskurve bis zum Maximum erstellt.

Potentielle Endothelin-Antagonisten werden an anderen Präparaten des gleichen Gefäßes 15 min vor Beginn der Endothelin-Dosiswirkungskurve appliziert. Die Effekte des Endothelins werden in % der K⁺-Kontraktur berechnet. Bei wirksamen Endothelin-Antagonisten kommt es zur Rechtsverschiebung der Endothelin-Dosiswirkungskurve.

Die erfindungsgemäßen Verbindungen können in üblicher Weise oral oder parenteral (subkutan, intravenös, intramuskulär, intraperitoneal) verabfolgt werden. Die Applikation kann auch mit Dämpfen oder Sprays durch den Nasen-Rachenraum erfolgen.

Die Dosierung hängt vom Alter, Zustand und Gewicht des Patienten sowie von der Applikationsart ab. In der Regel beträgt die tägliche Wirkstoffdosis zwischen etwa 0,5 und 50 mg/kg Körpergewicht bei oraler Gabe und zwischen etwa 0,1 und 10 mg/kg Körpergewicht bei parenteraler Gabe.

Die neuen Verbindungen können in den gebräuchlichen galenischen Applikationsformen fest oder flüssig angewendet werden, z.B. als Tabletten, Filmtabletten, Kapseln, Pulver, Granulate, Dragees, Suppositorien, Lösungen, Salben, Cremes oder Sprays. Diese werden in üblicher Weise hergestellt. Die Wirkstoffe können dabei mit den üblichen galenischen Hilfsmitteln wie Tablettenbindern, Füllstoffen, Konservierungsmitteln, Tablettensprengmitteln, Fließreguliermitteln, Weichmachern, Netzmitteln, Dispergiermitteln, Emulgatoren, Lösungsmitteln, Retardierungsmitteln, Antioxidantien und/oder Treibgasen verarbeitet werden (vgl. H. Sucker et al.: Pharmazeutische Technologie, Thieme-Verlag, Stuttgart, 1991). Die so erhaltenen Applikationsformen enthalten den Wirkstoff normalerweise in einer Menge von 0,1 bis 90 Gew.-%.

### Synthesebeispiele

### Beispiel 1

### 3,3-Bis(4-methoxyphenyl)butansäure

a) (2E,Z) 3-(4-Methoxyphenyl)but-2-ensäureethylester (6,6 g, 30 mmol) wurde bei 0°C in Anisol (4,9 g, 45 mmol) gelöst, und vorsichtig mit 50 ml 80 % H₂SO₄ versetzt. Das 2-Phasengemisch wurde 20 h stark bei Raumtemperatur gerührt, dann auf Eis gegossen und das Produkt mit Essigsäureethylester extrahiert. Die organische Phase wurde getrocknet (Na₂SO₄) filtriert, eingeengt, der Rückstand in Ether aufgenommen, mit 2N Natronlauge extrahiert und die Etherphase verworfen. Die alkalische Phase brachte man mit 2N HCl auf pH 2 und extrahierte das Produkt mit Essigsäureethylester. Die organische Phase wurde nun getrocknet (Na₂SO₄), filtriert, eingeengt und der feste Rückstand mit Diisopropylether ausgerührt. Das Produkt wurde abgesaugt und getrocknet. Es verblieben 5,1 g eines weißen Pulvers (56 %).
   Schmelzpunkt: 161-164°C
   Die Mutterlauge konnte weiter aufgearbeitet werden, wobei nochmals 1,1 g (12 %) der Säure erhalten wurde. Alternativ läßt sich die Säure auch wie folgt herstellen:
b) Bei 0°C wurden 32 ml Anisol (294 mmol) mit 33 ml Acetessigsäureethylester (258 mmol) gemischt, vorsichtig mit 150 ml 70 % H₂SO₄ versetzt, und das so erhaltene 2-Phasengemisch 72 h bei Raumtemperatur stark gerührt. Danach wurde der Ansatz auf Eis gegossen und wie unter la) weiter aufgearbeitet. Der Rückstand wurde aus Diisopropylether umkristallisiert. Es verblieben 15,3 g (35 %) weißer Feststoff.
c) Analog zur Herstellung von 3,3-Diphenylbutansäure (Beispiel 3,4)

### Beispiel 2

### (2R,S)-3,3-Bis-(4-methoxyphenyl)-2-(3',4'-methylendioxybenzyl)-butansäure

Eine Lösung von Diisopropylamin (3,1 ml, 22 mmol) in 50 ml trockenem Tetrahydrofuran wurde unter Stickstoff bei -10°C mit Butyllithium (13,8 ml, 22 mmol, 1,6M in Hexan) versetzt, 5 min bei -10°C gerührt, und dann bei 0°C 3,3-Bis(4-methoxyphenyl)butansäure (3,0 g, 10 mmol) in 15 ml absolutem THF zugetropft. Nach beendeter Zugabe wurde 1 h bei Raumtemperatur gerührt auf -20°C abgekühlt, Piperonylbromid (2,6 g, 12 mmol) in 10 ml THF zugegeben und anschließend 72 h bei Raumtemperatur gerührt. Dann wurde der Ansatz mit gesättigter NH₄Cl-Lösung gequencht, die organische Phase abgetrennt und die wäßrige mit Essigsäureethylester extrahiert. Die vereinigten organischen Extrakte wurden getrocknet (Na₂SO₄), filtriert, und am Rotationsverdampfer eingeengt. Der braune Rückstand wurde an Kieselgel chromatographiert (Methanol/ CH₂Cl₂ 1:19), wobei 1,3 g (30 %) Produkt als weißer Schaum erhalten wurden.
Schmelzpunkt: 137-140°C (aus Diisopropylether)

### Beispiel 3

### 3,3-Diphenyl-butansäureethylester

Bei 0°C wurden 65 g AlCl₃ (487 mmol) in 500 ml Benzol suspendiert und langsam mit 61,7 g (2E,Z) 3-Phenyl-but-2-ensäureethylester versetzt. Die dunkelrote Lösung wurde 20 h bei Raumtemperatur gerührt und dann auf ein Gemisch Eis/conc. HCl gegossen. Die organische Phase wurde abgetrennt, die wäßrige mit Essigsäureethylester extrahiert. Die vereinigten organischen Phasen wurden mit NaOH extrahiert, dann getrocknet (Na₂SO₄), filtriert und eingeengt (66,8 g dunkelbraunes Öl).
56,5 g dieses Öls wurden destilliert wobei 46,3 g Produkt als farbloses Öl erhalten wurden.

### Beispiel 4

### 3,3-Diphenyl-butansäure

In 30 ml Dioxan wurden 4,9 g 3,3-Diphenylbutansäureethylester (18,3 mmol) gelöst, mit 36 ml 1M KOH versetzt und 6 h bei 60-70°C gerührt.

Dann wurde das Dioxan am Rotationsverdampfer abgezogen, der wäßrige Rückstand mit Wasser verdünnt und mit Diethylether extrahiert. Anschließend wurde die wäßrige Phase auf pH 1 gebracht und mit Essigsäureethylester extrahiert. Die organischen Phase wurde getrocknet (Na₂SO₄), filtriert und eingeengt. Den festen Rückstand rührte man mit Heptan aus, und man erhielt 2,35 g eines weißen Pulvers (55 %). Die Mutterlauge wurde nicht weiter aufgereinigt.

### Beispiel 5

### (2R,S) 3,3-Diphenyl-2-(3',4'-methylendioxybenzyl)butansäure

Zu einer Lösung von 3,3-Diphenylbutansäure (2,4 g, 10 mmol) in 40 ml absolutem THF wurde bei -20°C 15 ml Butyllithium (24 mmol, 1,6M in Hexan) zugetropft, und anschließend 1 h zwischen -10 und -20°C gerührt. Dann gab man Piperonylchlorid (2,2 g, 13 mmol) in 10 ml THF zu, rührte 16 h bei Raumtemperatur und quenchte dann mit ges. NH₄Cl-Lösung. Die organische Phase wurde abgetrennt, die wäßrige Phase mit Essigsäureethylester extrahiert, dann die vereinigten organischen Extrakte getrocknet (Na₂SO₄), filtriert und eingeengt. Der Rückstand wurde an Kieselgel chromatographiert (CH₂Cl₂/MeOH 19:1), wobei man 2,4 g des gewünschten Produktes erhielt (65 %).

Die Säure wurde in CH₂Cl₂ gelöst und mit ges. Natriumcarbonatlösung ausgeschüttelt. Die organische (!) Phase wurde abgetrennt getrocknet (Na₂SO₄), filtriert und eingeengt. Man erhielt 2,5 g des Natriumsalzes der Säure.
Schmelzpunkt: 308-310°C (Zers.)

### Beispiel 6

### 3,3-Bis(4-methoxy-3-methylphenyl)butansäure

Die Herstellung erfolgte analog zu Beispiel 1b. In diesem Fall wurde jedoch hauptsächlich der entsprechende Ethylester isoliert, so daß noch eine nachträgliche Verseifung nötig war (analog Bsp. 4).
Schmelzpunkt: 121-124°C

### Beispiel 7

### (2R,S) 3,3-Bis(4-methoxy-3-methylphenyl)-2-(3',4'-methylendioxybenzyl)butansäure

### Herstellung analog zu Beispiel 2.

3,25 ml Diisopropylamin (23 mmol), 15,6 ml Butyllithium (23 mmol, 1,5 M in Hexan), 3,28 g 3,3-Bis (4-methoxy-3-methylphenyl)butansäure (10 mmol), 2,19 g Piperonylchlorid (13 mmol), ergeben 4,1 g Rohprodukt.
Chromatographie an Kieselgel (CH₂Cl₂/MeOH 19:1) ergaben 1,6 g Produkt (35 %)
Schmelzpunkt: 152-153°C

### Beispiel 8

### (2R,S) 3,3-Diphenyl-2-(3',4'-dimethoxybenzyl)butansäure

Die Herstellung erfolgte analog zu Beispiel 5. 2,4 g 3,3-Diphenylbutansäure (10 mmol), 15,6 ml Butyllithium (23 mmol, 1,5 M in Hexan, 2,2 g 3,4-Dimethoxybenzylchlorid (13 mmol) ergaben 3,8 g Rohprodukt.
Reinigung an Kieselgel (Heptan/Essigsäureethylester 1:1) 2,1 g Produkt (54 %)
Schmelzpunkt: 141-143°C

### Beispiel 9

3,3 Bis-(4-methoxyphenyl)pentansäure (2E,Z) 3-(4-Methoxyphenyl)pent-2-ensäureethylester (7,0 g, 30 mmol) wurde bei 0°C in Anisol (4,9 g, 45 mmol) gelöst und vorsichtig mit 50 ml 80 % H₂SO₄ versetzt. Das 2-Phasengemisch wurde 30 h stark bei Raumtemperatur gerührt, dann auf Eis gegossen und das Produkt mit Methylenchlorid extrahiert. Die organische Phase wurde getrocknet (Na₂SO₄), filtriert, eingeengt, der Rückstand in Ether aufgenommen, mit 2N Natronlauge extrahiert und die Etherphase verworfen. Die alkalische Phase brachte man mit 2N HCl auf pH 2 und extrahierte das Produkt mit Essigsäureethylester. Die organische Phase wurde nun getrocknet (Na₂SO₄), filtriert, eingeengt und der feste Rückstand mit Heptan ausgerührt. Das Produkt wurde abgesaugt und getrocknet. Es verblieben 6,8 g eines weißen Pulvers (72 %).
Schmelzpunkt: 136-139°C

### Beispiel 10

### (2R,S) 3,3 Bis-(4-methoxyphenyl)-2-(3',4'-methylendioxybenzyl) butansäure

Zu einer Lösung von 3,3-Bis-(4-methoxyphenyl)pentansäure (6,2 g, 20 mmol) in 100 ml absolutem THF wurden bei -20°C 29 ml Butyllithium (46 mmol, 1,6 M in Hexan) zugetropft und anschließend 1 h bei Raumtemperatur gerührt. Dann gab man bei -10°C Piperonylchlorid (4,4 g, 24 mmol) in 10 ml THF zu, rührte 72 h bei Raumtemperatur und quenchte dann mit ges. NH₄Cl-Lösung. Die organische Phase wurde abgetrennt, die wäßrige Phase mit Essigsäureethylester extrahiert, dann die vereinigten organischen Extrakte getrocknet (Na₂SO₄), filtriert und eingeengt. Der Rückstand (11,2 g) wurde an Kieselgel chromatographiert (CH₂Cl₂/MeOH 24:1), wobei man 3,1 g des gewünschten Produktes erhielt (34 %).
Schmelzpunkt: 84-86°C (ausgerührt in Heptan)

### Beispiel 11

### 3,3-Bis-(4-methoxyphenyl)hexansäuremethylester

Anisol (6,6 g, 61 mmol) wurden in 200 ml Dichlorethan gelöst, bei 0°C Aluminiumtrichlorid (12,3 g, 92 mmol) portionsweise zugegeben und anschließend unter Rühren (2E,Z) 3-(4-Methoxyphenyl)hex-2-ensäuremethylester (18 g, 61 mmol) zugetropft. Die Reaktionsmischung wurde 2 h bei 5°C und dann 2 Tage bei Raumtemperatur nachgerührt. Zur Aufarbeitung wurde die Mischung auf Eiswasser gegossen, mit CH₂Cl₂ extrahiert, die vereinigten organischen Phasen mit ges. NaCl-Lösung gewaschen und über MgSO₄ getrocknet. Der nach dem Einengen verbliebene Rückstand wurde durch Chromatographie an Kieselgel (n-Heptan/Essigsäureethylester 7,5 %) gereinigt. Auf diese Weise wurden 5,2 g (25 %) eines farblosen Öls erhalten.
¹H-NMR (CDCl₃), δ: 0,9 (m, 3H), 1,1 und 2,2 (je m, 2H), 3,08 (s, 2H), 3,4 (s, 3H) (s, 6H), 6,8 und 7,1 (je m, 4H) ppm.

### Beispiel 12

### 3,3-Bis-(4-methoxyphenyl)hexansäure

3,3-Bis-(4-methoxyphenyl)hexansäuremethylester (5,2 g, 15,2 mmol) wurde in 20 ml Dioxan vorgelegt, KOH (1,05 g, 18,2 mmol) zugegeben und ca. 1 h gekocht. Die Mischung wurde anschließend mit Wasser verdünnt, mit Essigsäureethylester gewaschen, die wäßrige Phase anschließend mit verd. HCl auf pH 3 gestellt und mit Essigsäureethylester extrahiert. Die organische Phase wurde dann mit ges. NaCl-Lösung gewaschen, über MgSO₄ getrocknet und eingeengt. Nach Chromatographie an Kieselgel (CH₂Cl₂/Methanol 3 %) wurden 4,1 g eines leicht gelblichen Öls erhalten (84 %).
¹H-NMR (CDCl₃), δ: 0,9 (m, 3H), 1,1 und 2,2 (je m, 2H), 3,1 (s, 3H), 3,8 (s, 6H), 6,8 und 7,1 je m, 4H) ppm.

### Beispiel 13

Die folgenden Verbindungen wurden analog zu Beispiel 5 hergestellt.

(2R,S) 3,3-Diphenyl-2-(methylnaphth-2'-yl)butansäure Schmelzpunkt: 163-166°C
FAB-MS: 380 (M⁺)

(2R,S) 3,3-Diphenyl-2-(3',5'-dimethylbenzyl)butansäure Schmelzpunkt: 141-143°C
FAB-MS: 358 (M⁺)

(2R,S) 3,3-Diphenyl-2-(4'-benzyloxy-3'-methoxybenzyl)butansäure Schmelzpunkt: 163-166°C
FAB-MS: 466 (M⁺)

(2R,S) 3,3-Bis-(4-methoxyphenyl)-2-(4'-benzyloxy-3-methoxybenzyl)butansäure
Schmelzpunkt: 137-140°C
FAB-MS: 526 (M⁺)

(2R,S) 3,3-Diphenyl-2-(4'-hydroxy-3'-methoxybenzyl)butansäure Schmelzpunkt: 153-155°C
FAB-MS: 376 (M⁺)

(2R,S) 3,3-Bis-(4-methoxyphenyl)-2-(4'-hydroxy-3-methoxybenzyl)-butansäure
Schmelzpunkt: 157-160°C
FAB-MS: 436 (M⁺)

(2R,S) 3,3-Bis-(4-methoxy-3-methylphenyl)-2-(3',5'-dimethylbenzyl)butansäure
Schmelzpunkt: 150-152°C
FAB-MS: 446 (M⁺)

(2R,S) 3,3-Bis-(4-methoxyphenyl)-2-(methylnaphth-2'-yl)butansäure Schmelzpunkt: 162-164°C
FAB-MS: 440 (M⁺)

(2R,S) 3,3-Bis-(4-methoxyphenyl)-2-(3',5'-dimethylbenzyl)butansäure
Schmelzpunkt: 125-128°C
FAB-MS: 418 (M⁺)

(2R,S) 3,3-Bis-(4-methoxy-3-methylphenyl)-2- (3',4'-dimethoxybenzyl)butansäure
Schmelzpunkt: 155-157°C
FAB-MS: 478 (M⁺)

(2R,S) 3,3-Bis-(4-methoxyphenyl)-2-(3',4'-dimethoxybenzyl)butansäure
Schmelzpunkt: 148-150°C
FAB-MS: 450 (M⁺)

(2R,S) 3,3-Bis-(4-methoxyphenyl)-2-(5'-methoxy-3',4'-methylendioxybenzyl)pentansäure
Schmelzpunkt: 138-141°C
FAB-MS: 478 (M⁺)

(2R,S) 3,3-Bis-(4-methoxyphenyl)-2-(5'-methoxy-3',4'-methylendioxybenzyl)butansäure
Schmelzpunkt: 134-136°C
FAB-MS: 464 (M⁺)

(2R,S) 3,3-Diphenyl-2-(5'-methoxy-3',4'-methylendioxybenzyl)-butansäure
Schmelzpunkt: 135-138°C
FAB-MS: 464 (M⁺)

(2R,S) 3,3-Bis-(4-methoxyphenyl)-2-(3',4'-ethylendioxybenzyl)-pentansäure
Schmelzpunkt: 168-170°C
FAB-MS: 462 (M⁺)

(2R,S) 3,3-Bis-(4-methoxyphenyl)-2-(3',4'-ethylendioxybenzyl)-butansäure
Schmelzpunkt: 161-163°C
FAB-MS: 448 (M⁺)

(2R,S) 3,3-Bis-(4-methoxyphenyl)-2-(3',4'-methylendioxybenzyl)-hexansäure
Schmelzpunkt: 142-145°C (aus n-Heptan)

(2R,S) 3,3-Bis-(4-methoxyphenyl)-2-(3',4'-ethylendioxybenzyl)-hexansäure
Schmelzpunkt: 163-165°C (aus n-Heptan/Diethylether)

(2R,S) 3,3-Bis-(4-methoxyphenyl)-2-(3',4'-methylendioxy-5'methoxybenzyl)hexansäure
Schmelzpunkt: 180-182°C (aus n-Heptan/Diethylether)

### Beispiel 14

Die in den Beispielen 2 bis 10 hergestellten Verbindungen wurden gemäß den oben beschriebenen Verfahren auf ihre Endothelinrezeptor-Affinität überprüft. Als Vergleichssubstanz wurde eine aus WO 94/02474 bekannte Verbindung verwendet. Das Ergebnis ist in der folgenden Tabelle beschrieben.

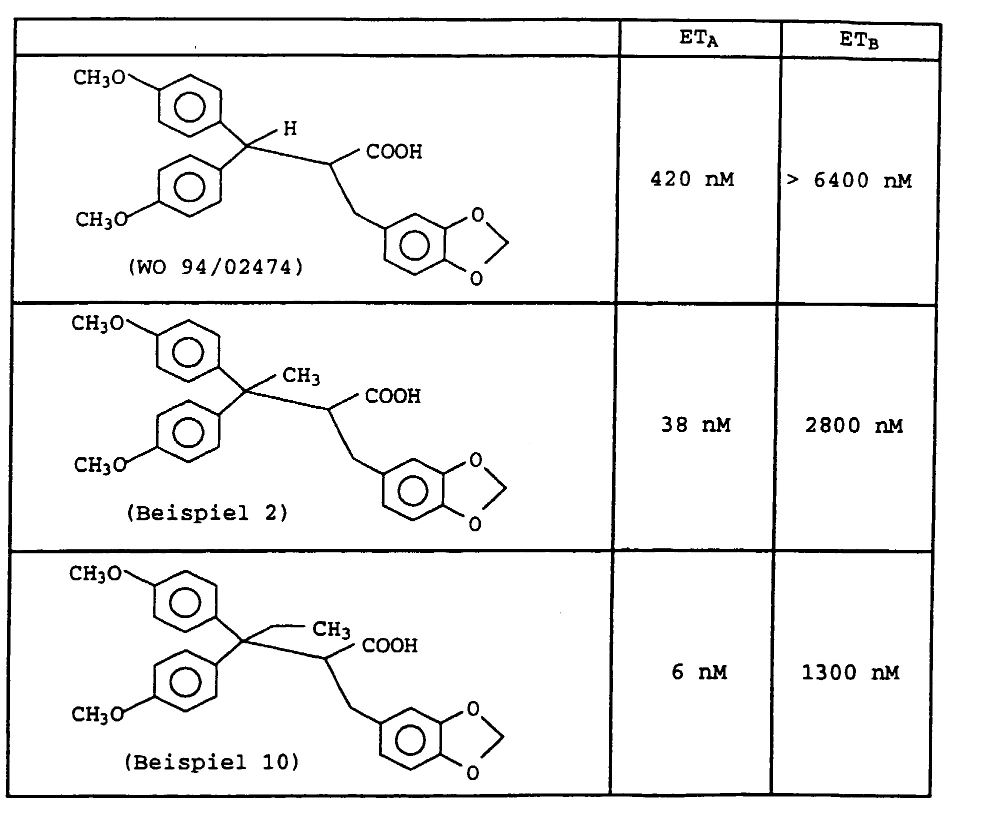

## Patentansprüche

1. Carbonsäurederivate der Formel I 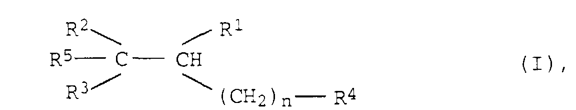 in der R¹ ein Tetrazol, Nitril, eine Gruppe COOH oder einen zu COOH hydrolysierbaren Rest bedeutet und die übrigen Substituenten folgende Bedeutung haben:
R² und R³ (die gleich oder verschieden sein können):
Phenyl oder Naphthyl, die durch einen oder mehrere der folgenden Reste substituiert sein können: Halogen, Cyano, NO₂, Hydroxy, C₁-C₄-Alkyl, C₁-C₄-Halogenalkyl, C₁-C₄-Alkoxy, C₁-C₄-Kalogenalkoxy, Phenoxy, C₁-C₄-Alkylthio, Amino, Benzyloxy, C₁-C₄-Alkylamino oder C₁-C₄-Dialkylamino; oder
Phenyl oder Naphthyl, die orthoständig über eine direkte Bindung, eine Methylen-, Ethylen- oder Ethenylengruppe, ein Sauerstoff- oder Schwefelatom miteinander verbunden sind;
R⁴ Phenyl oder Naphthyl, Methylendioxyphenyl, Ethylendioxyphenyl, indanyl, Indolyl, Pyridyl, Benzopyranyl, Furanyl, Pyrimidinyl, Benzofuranyl, Isooxazolyl, Isothiazolyl, 1,3,4-Thiadiazolyl, 2,3-Dihydrobenzofuranyl, Benzothienyl, Chinolinyl, C₃-C₇-Cycloalkyl, Thienyl, Oxazolyl, Thiazolyl, die durch einen oder mehrere der folgenden Reste substituiert sein können: Halogen, Cyano, Hydroxy, NO₂, C₁-C₄-Alkyl, C₁-C₄-Halogenalkyl, C₁-C₄-Alkoxy, C₁-C₄-Halogenalkoxy, Phenoxy, C₁-C₄-Alkylthio, Amino, Benzyloxy, C₁-C₄-Alkylamino oder C₁-C₄-Dialkylamino, wobei die Alkyreste gemeinsam einen Ring bilden können;
R⁵ C₁-C₈-Alkyl, C₃-C₆-Alkenyl, C₃-C₆-Alkinyl oder C₃-C₈-Cycloalkyl, wobei diese Reste jeweils ein- oder mehrfach substituiert sein können durch: Halogen, C₁-C₄-Alkoxy, C₁-C₄-Alkylthio, C₁-C₄-Alkylamino, Di-C₁-C₄-alkylamino;
Phenyl, Benzyl, 1-Methylnaphthyl, 2-Methylnaphthyl oder Naphthyl, die jeweils durch einen oder mehrere der folgenden Reste substituiert sein können: Halogen,
Cyano, Hydroxy, Amino, C₁-C₄-Alkyl, C₁-C₄-Alkoxy, Phenoxy, C₁-C₄-Alkylthio, Dioxomethylen oder Dioxoethylen;
n 1 - 2;

2. Verwendung von Carbonsäurederivaten gemäß Anspruch 1 zur Herstellung von Arzneimitteln.

## Claims

1. A carboxylic acid derivative of the formula I 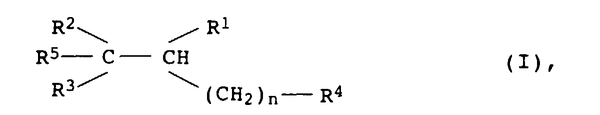 where R¹ is tetrazolyl, cyano, COOH or a radical which can be hydrolyzed to COOH, and the other substituents have the following meanings:
R² and R³ (which can be identical or different):
phenyl or naphthyl which can be substituted by one or more of the following radicals: halogen, cyano, NO₂, hydroxyl, C₁-C₄-alkyl, C₁-C₄-haloalkyl, C₁-C₄-alkoxy, C₁-C₄-haloalkoxy, phenoxy, C₁-C₄-alkylthio, amino, benzyloxy, C₁-C₄-alkylamino or C₁-C₄-dialkylamino; or
phenyl or naphthyl which are connected together in the ortho positions by a direct linkage, a methylene, ethylene or ethenylene group, or an oxygen or sulfur atom;
R⁴ phenyl or naphthyl, methylenedioxyphenyl, ethylenedioxyphenyl, indanyl, indolyl, pyridyl, benzopyranyl, furanyl, pyrimidinyl, benzofuranyl, isooxazolyl, isothiazolyl, 1,3,4-thiadiazolyl, 2,3-dihydrobenzofuranyl, benzothienyl, quinolinyl, C₃-C₇-cycloalkyl, thienyl, oxazolyl, thiazolyl, each of which can be substituted by one or more of the following radicals: halogen, cyano, hydroxyl, NO₂, C₁-C₄-alkyl, C₁-C₄-haloalkyl, C₁-C₄-alkoxy, C₁-C₄-haloalkoxy, phenoxy, C₁-C₄-alkylthio, amino, benzyloxy, C₁-C₄-alkylamino or C₁-C₄-dialkylamino, it being possible for the alkyl radicals together to form a ring;
R⁵ C₁-C₈-alkyl, C₃-C₆-alkenyl, C₃-C₆-alkynyl or C₃-C₈-cycloalkyl, it being possible for each of these radicals to be substituted one or more times by: halogen, C₁-C₄-alkoxy, C₁-C₄-alkylthio, C₁-C₄-alkylamino, di-C₁-C₄-alkylamino;
phenyl, benzyl, 1-methylnaphthyl, 2-methylnaphthyl or naphthyl, each of which can be substituted by one or more of the following radicals: halogen, cyano, hydroxyl, amino, C₁-C₄-alkyl, C₁-C₄-alkoxy, phenoxy, C₁-C₄-alkylthio, methylenedioxy or ethylenedioxy;
n 1 - 2.

2. The use of a carboxylic acid derivative as claimed in claim 1 for producing drugs.

## Revendications

1. Dérivés d'acide carboxylique de formule I 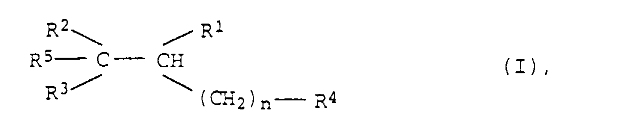 dans laquelle R¹ représente un tétrazole, un nitrile, un groupe COOH ou un reste hydrolysable en COOH et les autres substituants ont la signification suivante:
R² et R³ (qui peuvent être identiques ou différents):
phényle ou naphtyle, qui peuvent être substitués par un ou plusieurs des restes suivants: halogéno, cyano, NO₂, hydroxy, alkyle en C₁-C₄, halogénoalkyle en C₁-C₄, alcoxy en C₁-C₄, halogénoalcoxy en C₁-C₄, phénoxy, alkylthio en C₁-C₄, amino, benzyloxy, alkylamino en C₁-C₄ ou dialkyl(C₁-C₄)amino; ou
phényle ou naphtyle, qui sont liés entre eux en position ortho par une liaison directe, un groupe méthylène, éthylène ou éthényle, un atome d'oxygène ou un atome de soufre;
R⁴ phényle ou naphtyle, méthylènedioxyphényle, éthylènedioxyphényle, indanyle, indolyle, pyridyle, benzopyrannyle, furannyle, pyrimidinyle, benzofurannyle, isooxazolyle, isothiazolyle, 1,3,4-thiadiazolyle, 2,3-dihydobenzofurannyle, benzothiényle, quinoléinyle, cycloalkyle en C₃-C₇, thiényle, oxazolyle, thiazolyle, qui peuvent être substitués par un ou plusieurs des restes suivants: halogéno, cyano, hydroxy, NO₂, alkyle en C₁-C₄, halogénoalkyle en C₁-C₄, alcoxy en C₁-C₄, halogénoalcoxy en C₁-C₄, phénoxy, alkylthio en C₁-C₄, amino, benzyloxy, alkylamino en C₁-C₄ ou dialkyl(C₁-C₄)amino, tandis que les restes alkyle peuvent former un cycle ensemble;
R⁵ alkyle en C₁-C₈, alcényle en C₃-C₆, alcynyle en C₃-C₆ ou cycloalkyle en C₃-C₈, tandis que les restes peuvent être substitués chacun une ou plusieurs fois par: halogéno, alcoxy en C₁-C₄, alkylthio en C₁-C₄, alkylamino en C₁-C₄, dialkyl(C₁-C₄)amino;
phényle, benzyle, 1-méthylnaphtyle, 2-méthylnaphtyle ou naphtyle, qui peuvent être substitués chacun par un ou des plusieurs restes suivants: halogéno, cyano, hydroxy, amino, alkyle en C₁-C₄, alcoxy en C₁-C₄, phénoxy, alkylthio en C₁-C₄, dioxométhylène ou dioxoéthylène;
n 1 - 2.

2. Utilisation de dérivés d'acide carboxylique selon la revendication 1 pour la préparation de médicaments.
